# EUROPEAN PATENT APPLICATION

(11) **EP 4 353 811 A2**
(43) Date of publication of application: **17.04.2024**
(21) Application number: 23202087.5
(22) Date of filing: 06.10.2023
(51) Int. Cl.: C12M 1/00

(54) **ENZYME REACTOR**

(30) Priority: 11.10.2022 JP 2022163140
(71) Applicant: Hitachi, Ltd., Tokyo 100-8280 (JP)
(72) Inventor: NISHIDA, Hirokazu, Toyko, 100-8280 (JP)
(74) Representative: MERH-IP Matias Erny Reichl Hoffmann Patentanwälte PartG mbB

(57) **Abstract**

Provided is an enzyme reactor (100) that allows producing a high valueadded substance by performing a process in which a reduction reaction and an enzymatic reaction are linked using energy generated by a photocatalyst. An enzyme reactor (100) according to the present invention includes: an ion exchange membrane (4) that partitions an inside of a reaction vessel (2) into an anode chamber (2A) and a cathode chamber (2C); a semipermeable membrane (6) that partitions the cathode chamber (2C) into a photocatalytic reaction chamber (2P) and an enzymatic reaction chamber (2E); an anodic electrolytic solution (LA) housed in the anode chamber (2A); a cathodic electrolytic solution (LC) housed in the photocatalytic reaction chamber (2P); an enzyme reaction solution (LE) housed in the enzymatic reaction chamber (2E); an anodic electrode (10A) disposed in the anode chamber (2A); and a cathodic electrode (10C) disposed in the photocatalytic reaction chamber (2P). The cathodic electrode (10C) has a surface on which a photocatalyst layer (10Cp) is disposed. The cathodic electrolytic solution (LC) contains an electron mediator. The enzyme reaction solution (LE) contains an enzyme and a raw material substrate. The cathodic electrolytic solution (LC) and the enzyme reaction solution (LE) are in contact with one another via the semipermeable membrane (6). The semipermeable membrane (6) allows the reduced electron mediator to permeate through and does not allow the enzyme to permeate through.

## Description

### Technical Field

The present invention relates to an enzyme reactor and, more particularly, to the enzyme reactor for performing an enzymatic reaction by using energy generated by irradiating a photocatalyst with light.

### Background Art

The elevated levels of greenhouse gases in the atmosphere are thought to be a major cause of global warming. In particular, carbon dioxide (CO₂), whose emissions have increased exponentially in the last two centuries, is a major emission control target. The global warming and the associated disasters caused by the climate change have already become a problem, and fixation (absorption) of carbon dioxide released into the air in parallel with the emission control is also an urgent problem. Various methods of fixing carbon dioxide have been proposed so far, but few of them can be operated at low cost and low energy. As a method of fixing carbon dioxide, it is desirable to convert the value of carbon dioxide into other chemical products and return the value to society. In recent years, advances in a synthetic biotechnology have made it possible to use various compounds including carbon dioxide as raw materials to produce substances with high biochemical added value. However, at present, reaction systems that enable the fixation of carbon dioxide and the production of high value-added substances are under development.

The photocatalyst, such as a transition metal oxide, absorbs light energy when irradiated with light, and generates the holes on its surface to emit the electrons. As the photocatalyst, for example, titanium oxide which is widely used as an antibacterial substance by performing sterilization or decomposition of an organic substance by these actions is known. For example, the transition metal oxide such as titanium oxide, which is a material of the photocatalyst, has a large number of combinations due to differences in the type, number, oxidation number, etc. of the transition metals. In addition, even when the transition metal oxides have the same chemical composition, the crystal and quasi-crystal structure of the transition metal oxides have thousands of variations, and the search for a transition metal oxide that receives light like titanium oxide and thereby causes a reaction having high utilization value on its surface has just started. As for the photocatalyst made of a material such as a transition metal oxide, it is expected that a photocatalyst that contributes to a reduction reaction that leads to the production of various useful substances will be developed in the future. Specifically, among the photocatalysts, the photocatalysts that can directly reduce the carbon dioxide and the photocatalysts that can reduce the electron mediator and the like, which are energy sources in the synthetic bioprocesses that produce high value-added substances, are expected to play very significant roles as energy sources in the decarbonization processes in the future, its development is especially desired.

It is believed that carbon dioxide, which currently is contained only a small percentage by about 0.04 v/v% in the atmosphere, was present in the primitive atmosphere 4.6 billion years ago at the same level as nitrogen when the Earth was considered to have emerged. Approximately 2.7 billion years ago, it is thought that appearance of cyanobacteria that perform a photosynthesis began to reduce carbon dioxide in the atmosphere, and oxygen began to increase. The standard enthalpy of formation of carbon dioxide (hereinafter sometimes abbreviated as "ΔfH°") is -394 kJ/mol, which is as stable as metallic oxides, whereas ΔfH° of oxygens is 0 kJ/mol. Although 2.7 billion years of the plant photosynthetic energy production activities have led to large potential chemical bond energies in the atmosphere, the human production activities have resulted in compositional changes in the atmosphere that have led to the climate change, particularly during the last two centuries.

On the other hand, the reduction of carbon dioxide can restore the chemical bond energy which has been consumed since the industrial revolution, thus the progress of global warming can be suppressed. Furthermore, since the electron mediator is reduced to become an energy source in a process such as a synthetic bioprocess, the reduction of the electron mediator may allow the creation of a process for producing a substance such as an organic substance independently of energy derived from the fossil fuels. Therefore, generalization, functional improvement, and multipurpose of the photocatalyst have the possibility of greatly changing the standard which is demanded at present of the production process of the substance.

### Summary of Invention

### Technical Problem

From the above, it is desired to effectively combine the generalization, functional improvement, and multipurpose of the photocatalyst; and the production process for the substance having high added value. Specifically, there is a demand for a device capable of linking a photocatalytic reaction having an effective reducing ability for an intended substance and an enzymatic reaction such as a biochemical reaction in which a substance reduced by a photocatalytic reaction is used to produce a substance having a high added value.

In contrast, studies have been conducted on the reduction of substances such as carbon dioxide and the electron mediator by photocatalysts, but the number of examples is small. In addition, there has not been established a method in which a reduction of a substance such as carbon dioxide or the electron mediator by photocatalysts and a substance generation (substance conversion) by an enzymatic reaction such as a biochemical reaction are linked in a single reaction vessel to be performed.

The present invention has been made in view of the above problems, and an object of the present invention is to provide an enzyme reactor capable of producing a high value-added substance by performing a process in which a reduction reaction for reducing a substance and an enzymatic reaction for producing a substance using the substance reduced by the reduction reaction are linked by using energy generated by irradiating a photocatalyst with light.

### Solution to Problem

In order to solve the above problem, an enzyme reactor according to the present invention includes: a reaction vessel; an ion exchange membrane that partitions an inside of the reaction vessel into an anode chamber and a cathode chamber; a semipermeable membrane that partitions the cathode chamber into a photocatalytic reaction chamber and an enzymatic reaction chamber; an anodic electrolytic solution housed in the anode chamber; a cathodic electrolytic solution housed in the photocatalytic reaction chamber; an enzyme reaction solution housed in the enzymatic reaction chamber; an anodic electrode disposed in the anode chamber so as to be immersed in the anodic electrolytic solution; and a cathodic electrode disposed in the photocatalytic reaction chamber so as to be immersed in the cathodic electrolytic solution. The cathodic electrode has a surface on which a photocatalyst layer containing a photocatalyst is disposed. The cathodic electrolytic solution contains an electron mediator. The enzyme reaction solution contains an enzyme and a raw material substrate. The cathodic electrolytic solution and the enzyme reaction solution are in contact with one another via the semipermeable membrane. The semipermeable membrane allows the reduced electron mediator to permeate through the semipermeable membrane and does not allow the enzyme to permeate through the semipermeable membrane.

With the enzyme reactor of the present invention, the use of the energy generated by irradiating the photocatalyst with light allows performing a process in which a reduction reaction for reducing the electron mediator and an enzymatic reaction for producing a substance using the electron mediator reduced by the reduction reaction are linked, which produces a high value-added substance.

An enzyme reactor according to the present invention includes a reaction vessel; an ion exchange membrane that partitions an inside of the reaction vessel into an anode chamber and a cathode chamber; a semipermeable membrane that partitions the cathode chamber into a photocatalytic reaction chamber and an enzymatic reaction chamber; an anodic electrolytic solution housed in the anode chamber; a cathodic electrolytic solution housed in the photocatalytic reaction chamber; an enzyme reaction solution housed in the enzymatic reaction chamber; an anodic electrode disposed in the anode chamber so as to be immersed in the anodic electrolytic solution; and a cathodic electrode disposed in the photocatalytic reaction chamber so as to be immersed in the cathodic electrolytic solution. The cathodic electrode has a surface on which a photocatalyst layer containing a photocatalyst is disposed. The cathodic electrolytic solution contains carbon dioxide. The enzyme reaction solution contains an enzyme. The cathodic electrolytic solution and the enzyme reaction solution are in contact with one another via the semipermeable membrane. The semipermeable membrane allows the reduced carbon dioxide to permeate through the semipermeable membrane and does not allow the enzyme to permeate through the semipermeable membrane.

With the enzyme reactor of the present invention, the use of the energy generated by irradiating the photocatalyst with light allows performing a process in which a reduction reaction for reducing the carbon dioxide and an enzymatic reaction for producing a substance using the carbon dioxide reduced by the reduction reaction are linked, which produces a high value-added substance.

### Advantageous Effects of Invention

With the enzyme reactor of the present invention, the use of the energy generated by irradiating the photocatalyst with light allows performing a process in which a reduction reaction for reducing a substance and an enzymatic reaction for generating a substance using the substance reduced by the reduction reaction are linked, which produces a high value-added substance.

Further, with the enzyme reactor of the present invention, a process can be constructed by sequentially combining methods for producing a substance from a raw material substrate by linking and performing a reduction reaction and an enzymatic reaction using the enzyme reactor for a process for synthesizing a high value-added molecule in all the conventional methods for producing petrochemical products and a process for biochemical synthesis of a high value-added molecule in the fermentation industry. Further, since it is possible to accelerate the determination process for determining whether or not a process in which a reduction reaction and an enzymatic reaction are linked has been performed, the synthesis process of the molecule can be searched at a lower cost and rationally.

In addition, among the enzyme reactors of the present invention, in the enzyme reactor in which the cathodic electrolytic solution contains carbon dioxide, since the cathodic electrolytic solution contains another greenhouse gas instead of carbon dioxide, a reduction reaction for reducing the other greenhouse gas in the cathodic electrolytic solution and an enzymatic reaction for producing another substance from substances (the reduced substances) which is obtained by reducing the other greenhouse gas in the enzyme reaction solution are allowed to be linked and performed. Therefore, it is possible to search for an optimum condition for performing a process in which a reduction reaction and an enzymatic reaction are linked in order to produce a high value-added substance from the other greenhouse gas. In addition, it is possible to accelerate the determination process for determining whether or not a process in which a reduction reaction and an enzymatic reaction are linked has been able to be performed in order to generate the high value-added substance from the other greenhouse gas.

The problems, configurations, and effects of the present invention other than those described above will become apparent from the following description of embodiments of the present invention.

### Brief Description of Drawings

Fig. 1 is a schematic cross-sectional view illustrating an enzyme reactor of a first example according to a first embodiment.
Fig. 2 is a schematic cross-sectional view illustrating an enzyme reactor of a second example according to the first embodiment.
Fig. 3 is a schematic cross-sectional view illustrating an enzyme reactor of a third example according to the first embodiment.
Fig. 4 is a schematic cross-sectional view illustrating an enzyme reactor of a fourth example according to a first embodiment.
Fig. 5A is a photograph illustrating an overall outline of the enzyme reactor of Example 1.
Fig. 5BA is a top view schematically illustrating a main part of the enzyme reactor of Example 1.
Fig. 5BB is a cross-sectional view schematically illustrating a main part of the enzyme reactor of Example 1 and is a diagram illustrating a cross-section taken along the A-A' line in Fig. 5BA.
Fig. 5BC is a cross-sectional view schematically illustrating a main part of the enzyme reactor of Example 1 and is a diagram illustrating a cross-section taken along the B-B' line in Fig. 5BA.
Fig. 5C is a photograph illustrating an aspect of a method for using the enzyme reactor of Example 1.
Fig. 5D is a photograph illustrating another aspect of the method for using the enzyme reactor of Example 1.
Fig. 5E is a photograph obtained by enlarging a part of the device photographed in the photograph of Fig. 5D.
Fig. 6A is a top view schematically illustrating a main part of the enzyme reactor of Example 2.
Fig. 6B is a cross-sectional view schematically illustrating a main part of the enzyme reactor of Example 2 and is a diagram illustrating a cross section taken along the A-A' line in Fig. 6A.
Fig. 6C is a cross-sectional view schematically illustrating a main part of the enzyme reactor of Example 2 and is a diagram illustrating a cross section taken along the B-B' line in Fig. 6A.

### Description of Embodiments

Hereinafter, the enzyme reactor according to the embodiments of the present invention will be described. The following description illustrates specific examples according to the present invention, and the present invention is not limited to the specific examples illustrated in these descriptions, and various modifications can be made by those skilled in the art within the scope of the technical idea disclosed in the present specification.

### A. First Embodiment

An enzyme reactor according to a first embodiment includes: a reaction vessel; an ion exchange membrane that partitions an inside of the reaction vessel into an anode chamber and a cathode chamber; a semipermeable membrane that partitions the cathode chamber into a photocatalytic reaction chamber and an enzymatic reaction chamber; an anodic electrolytic solution housed in the anode chamber; a cathodic electrolytic solution housed in the photocatalytic reaction chamber; an enzyme reaction solution housed in the enzymatic reaction chamber; an anodic electrode disposed in the anode chamber so as to be immersed in the anodic electrolytic solution; and a cathodic electrode disposed in the photocatalytic reaction chamber so as to be immersed in the cathodic electrolytic solution. The cathodic electrode has a surface on which a photocatalyst layer containing a photocatalyst is disposed. The cathodic electrolytic solution contains an electron mediator. The enzyme reaction solution contains an enzyme and a raw material substrate. The cathodic electrolytic solution and the enzyme reaction solution are in contact with one another via the semipermeable membrane. The semipermeable membrane allows the reduced electron mediator to permeate through the semipermeable membrane and does not allow the enzyme to permeate through the semipermeable membrane.

### (First Example)

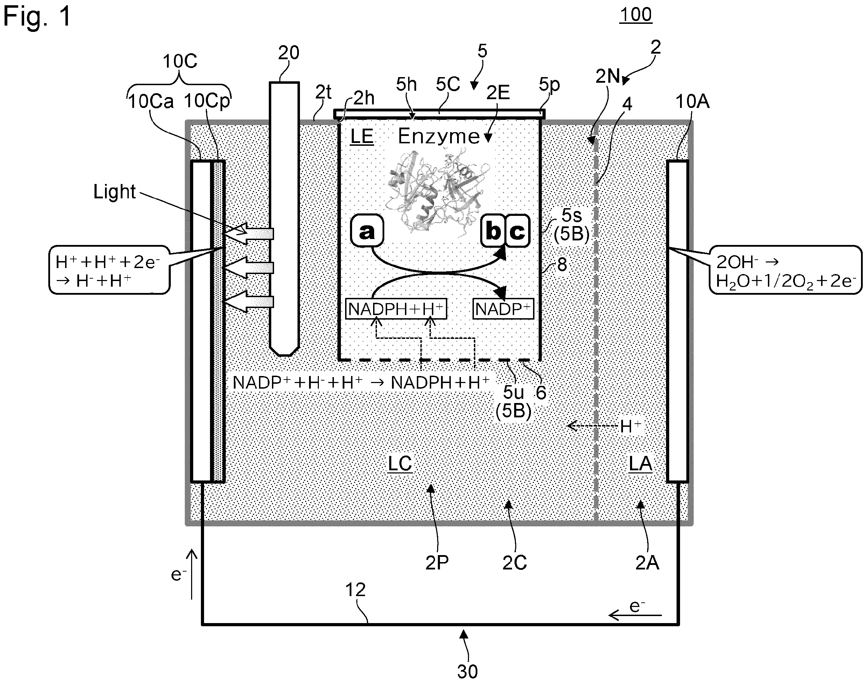

The enzyme reactor of a first example according to the first embodiment will be described. Fig. 1 is a schematic cross-sectional view illustrating the enzyme reactor of the first example according to the first embodiment.

First, a configuration of the enzyme reactor of the first example will be described.

As illustrated in Fig. 1, an enzyme reactor 100 of the first example includes a reaction vessel 2, a proton exchange membrane (ion exchange membrane) 4 that partitions an inside 2N of the reaction vessel 2 into an anode chamber 2A and a cathode chamber 2C, and a dialysis cup 5. The reaction vessel 2 includes a mounting port 2h having the same shape and size in plan view as those of a bottom portion 5u of a main body portion 5B of the dialysis cup 5 in shape and size in a plan view, and the mounting port 2h is provided at an upper portion 2t in a portion at a side of the cathode chamber 2C. The dialysis cup 5 includes the cylindrical main body portion 5B, which includes the bottom portion 5u and a side portion 5s and is provided with an opening portion 5h on an upper portion thereof facing the bottom portion 5u, and a cover portion 5C (which is closed in the figure) provided to allow opening and closing the opening portion 5h of the main body portion 5B. The cover portion 5C includes a protruding portion 5p protruding laterally with respect to the side portion 5s of the main body portion 5B. The dialysis cup 5 is mounted on the mounting port 2h of the reaction vessel 2 by inserting the main body portion 5B into the cathode chamber 2C through the mounting port 2h of the reaction vessel 2 and supporting the protruding portion 5p of the cover portion 5C at a periphery of the mounting port 2h in the upper portion 2t of the reaction vessel 2. The dialysis cup 5 can be mounted to and removed from the mounting port 2h of the reaction vessel 2. The bottom portion 5u of the main body portion 5B of the dialysis cup 5 is made of a semipermeable membrane 6, and the side portion 5s of the main body portion 5B is made of a non-permeable membrane 8. The cathode chamber 2C is partitioned into a photocatalytic reaction chamber 2P outside the dialysis cup 5 and an enzymatic reaction chamber 2E inside the dialysis cup 5 by the semipermeable membrane 6 of the bottom portion 5u and the non-permeable membrane 8 of the side portion 5s of the main body portion 5B of the dialysis cup 5.

The enzyme reactor 100 of the first example further includes an anodic electrolytic solution LA housed in the anode chamber 2A, a cathodic electrolytic solution LC housed in the photocatalytic reaction chamber 2P of the cathode chamber 2C, and an enzyme reaction solution LE housed in the enzymatic reaction chamber 2E of the cathode chamber 2C.

The anodic electrolytic solution LA is a predetermined electrolytic solution in which a predetermined electrolyte is dissolved in water. The cathodic electrolytic solution LC is obtained by dissolving NADP⁺, which is nicotinamide adenine dinucleotide phosphate (NADP) as an oxidation type electron mediator (oxidized electron mediator), in the common (same) electrolytic solution with the anodic electrolytic solution LA. The enzyme reaction solution LE is obtained by adding HMGR, which is 3-hydroxy-3-methylglutaryl coenzyme A reductase, as an enzyme, and HMG-CoA, which is 3-hydroxy-3-methylglutaryl-CoA, as a raw material substrate [a] to a common electrolytic solution with the anodic electrolytic solution LA. As the common electrolytic solution, specifically, for example, a mixed aqueous solution (buffer solution) of a weak acid or a weak base and a salt thereof, pH of which is adjusted within a range near an optimum pH of the enzyme (HMGR), and a temperature thereof is adjusted within a range near an optimum temperature of the enzyme (HMGR) is used.

The anodic electrolytic solution LA and the cathodic electrolytic solution LC are in contact with one another via the proton exchange membrane 4. The cathodic electrolytic solution LC and the enzyme reaction solution LE are in contact with one another via the semipermeable membrane 6. The semipermeable membrane 6 is a membrane that allows an oxidation type electron mediator (NADP⁺), a reduction type electron mediator (reduced electron mediator, NADPH) that is the oxidation type electron mediator (NADP⁺) having undergone the reduction, and protons (H⁺) to permeate through the membrane, and does not allow the enzyme (HMGR) to permeate through the membrane. It is preferred that the semipermeable membrane 6 be a membrane that allows an oxidation type electron mediator (NADP⁺), a reduction type electron mediator (NADPH), and the protons (H⁺) to permeate through the membrane and does not allow the enzyme (HMGR), the raw material substrate [a], a substance [b], and a substance [c] described later to permeate through the membrane. This is because formation of the substance [b] and the substance [c] is likely to occur, and a recovery of the substance [b] and the substance [c] is facilitated. However, it is difficult to make the semipermeable membrane 6 impermeable to the raw material substrate [a] when, for example, a molecular weight of the raw material substrate [a] is not higher than that of the electron mediator. When the semipermeable membrane 6 is not a membrane that does not allow the raw material substrate [a] to permeate through the membrane, the raw material substrate [a] becomes diffused into both the cathodic electrolytic solution LC and the enzyme reaction solution LE, but even in this state, a reduction reaction and a biochemical reaction (enzymatic reaction) can be linked and performed as described later. The non-permeable membrane 8 is a membrane that does not allow any component of the cathodic electrolytic solution LC or the enzyme reaction solution LE to permeate through the membrane.

The enzyme reactor 100 of the first example further includes an anodic electrode 10A arranged in the anode chamber 2A to be immersed in the anodic electrolytic solution LA and a cathodic electrode 10C arranged in the photocatalytic reaction chamber 2P to be immersed in the cathodic electrolytic solution LC. The cathodic electrode 10C includes a cathode substrate 10Ca and a photocatalyst layer 10Cp including a photocatalyst formed on a surface of the cathode substrate 10Ca. The enzyme reactor 100 of the first example further includes an external circuit 30 including a wiring 12. The wiring 12 electrically connects the anodic electrode 10A to the cathodic electrode 10C. The enzyme reactor 100 of the first example further includes a light source 20 and a power supply for light source (not illustrated). The light source 20 is disposed in the photocatalytic reaction chamber 2P such that the light source 20 is immersed in the cathodic electrolytic solution LC and faces the cathodic electrode 10C along a depth direction of the reaction vessel 2. The power supply for light source is disposed outside the reaction vessel 2 and connected to the light source 20. As the light source 20, specifically, a halogen lamp or a LED light having an electronically excited characteristic wavelength of the photocatalyst layer 10Cp is employed.

Next, an example of a method for producing a substance using the enzyme reactor 100 of the first example will be described.

In the example of the method for producing a substance using the enzyme reactor 100 of the first example, as illustrated in Fig. 1, first, electric power supplied from the power supply for light source to the light source 20 allows the light source 20 to irradiate the photocatalyst layer 10Cp on the cathodic electrode 10C with, for example, a light in a wavelength band from 350 nm to 3500 nm through the cathodic electrolytic solution LC. As a result, the excited electrons (e⁻) are generated on the photocatalyst layer 10Cp of the cathodic electrode 10C, and a reduction reaction represented in Reaction formula (1-1) below is performed in a vicinity of the cathodic electrode 10C in the cathodic electrolytic solution LC. In this reduction reaction, first, the protons (H⁺) contained in the cathodic electrolytic solution LC are reduced by the excited electrons (e⁻), hydride ions (H⁻) are generated, and then, an oxidation type electron mediator (NADP⁺) is reduced by the hydride ions (H⁻) to produce the reduction type electron mediator (NADPH). At the same time as the reduction reaction, an oxidizing reaction represented in Reaction formula (1-2) below is performed in a vicinity of the anodic electrode 10A in the anodic electrolytic solution LA. In this oxidation reaction, the hydroxide ions (OH⁻) lose the electrons, and water (H₂O) and oxygen (O₂) are produced.

NADP⁺ + 2H⁺ + 2e⁻ → NADP⁺ + H⁻ + H⁺ → NADPH + H⁺ (1-1)

2OH⁻ → H₂O + 1/2O₂ + 2e (1-2)

Next, as illustrated in Fig. 1, the reduction type electron mediator (NADPH) generated in the above-described reduction reaction and the protons (H⁺) remaining in the reduction reaction are transferred to the enzyme reaction solution LE of the enzymatic reaction chamber 2E by permeating through the semipermeable membrane 6.

Next, as described above, as a result of the transfer of the reduction type electron mediator (NADPH) and the protons (H⁺) to the enzyme reaction solution LE, the biochemical reaction (enzymatic reaction) illustrated in Reaction formula (1-3) below occurs in the enzyme reaction solution LE as illustrated in Fig. 1. In this biochemical reaction, the enzyme (HMGR) functions as a reductase, and the reduction type electron mediator (NADPH) functions as a coenzyme that imparts energy, thereby reducing the raw material substrate [a] (HMG-CoA). This produces mevalonate ((R)-mevalonate) and coenzyme A (CoA) as the substance [b] and the substance [c], respectively. At the same time, the reduction type electron mediator (NADPH) is oxidized to produce the oxidation type electron mediator (NADP⁺).

HMG-CoA + 2NADPH + 2H⁺ → (R)-mevalonate + CoA + 2NADP⁺ (1-3)

Next, although not illustrated, after the dialysis cup 5 is removed from the mounting port 2h of the reaction vessel 2, the cover portion 5C of the dialysis cup 5 is opened and the substance [b] (mevalonate) and the substance [c] (coenzyme A) are recovered from the enzyme reaction solution LE inside the dialysis cup 5. While the dialysis cup 5 is mounted on the mounting port 2h of the reaction vessel 2, the oxidation type electron mediator (NADP⁺) in the enzyme reaction solution LE inside the dialysis cup 5 is transferred to the cathodic electrolytic solution LC of the photocatalytic reaction chamber 2P by permeating through the semipermeable membrane 6, and is reduced again by the above-describe reduction reaction.

As described above, in the enzyme reactor 100 of the first example, the cathode chamber 2C is partitioned into the photocatalytic reaction chamber 2P and the enzymatic reaction chamber 2E by the semipermeable membrane 6 that allows the oxidation type electron mediator (NADP⁺), the reduction type electron mediator (NADPH), and the protons (H⁺) to permeate through the membrane 6 and does not allow the enzyme (HMGR) to permeate through the membrane 6. The photocatalytic reaction chamber 2P houses the cathodic electrolytic solution LC in which the oxidation type electron mediator (NADP⁺) is dissolved in the common electrolytic solution, and the enzymatic reaction chamber 2E houses the enzyme reaction solution LE in which the enzyme (HMGR) and the raw material substrate [a] (HMG-CoA) are added to the common electrolytic solution. For this reason, irradiation of the photocatalyst layer 10Cp of the cathodic electrode 10C with light allows the reduction reaction and a biochemical reaction (enzymatic reaction) to be linked and performed. In the reduction reaction, the oxidation type electron mediator (NADP⁺) is reduced by a reducing action with a photocatalyst in the cathodic electrolytic solution LC to form the reduction type electron mediator (NADPH). In the biochemical reaction (enzymatic reaction), the substance [b] (mevalonate) is generated from the raw material substrate [a] (HMG-CoA) using the reduction type electron mediator (NADPH) as a coenzyme in the enzyme reaction solution LE. Therefore, the use of the energy generated by irradiating the photocatalyst with light allows performing the process in which the reduction reaction for reducing the oxidation type electron mediator and the biochemical reaction (enzymatic reaction) for generating a substance using the reduction type electron mediator are linked, thus allowing the substance [b] having a high added value to be produced.

If the cathode chamber 2C were not partitioned by the semipermeable membrane 6 into the photocatalytic reaction chamber 2P and the enzymatic reaction chamber 2E and the entire cathode chamber 2C housed the cathodic electrolytic solution LC, in which the oxidation type electron mediator (NADP⁺) were dissolved in the electrolytic solution while the enzyme (HMGR) and the raw material substrate [a] (HMG-CoA) were added to the electrolytic solution, the enzyme would be attracted to a surface of the photocatalyst layer 10Cp and the enzyme would deteriorate due to a potential difference generated on the surface of the photocatalyst layer 10Cp when the photocatalyst layer 10Cp of the cathodic electrode 10C are irradiated with light, thereby losing its function.

In the enzyme reactor 100 of the first example, after the photocatalyst layer 10Cp of the cathodic electrode 10C is irradiated with light, the substance is recovered from the enzyme reaction solution LE inside the dialysis cup 5. Confirming whether or not the substance is the substance [b] (mevalonate) allows confirming whether or not a process in which the reduction reaction and the biochemical reaction are linked has been able to be performed. For this reason, it is possible to search for the optimum condition for performing the process in which the reduction reaction and the biochemical reaction are linked by determining whether or not the process in which the reduction reaction and the biochemical reaction are linked have been able to be performed while adjusting the conditions including the pH, temperature, etc. of the common electrolytic solution, the type of a solvent and the type, concentration, etc. of an electrolyte and another solute in the common electrolytic solution, the type, etc. of the photocatalyst layer, the type, concentration, etc. of the electron mediator in the cathodic electrolytic solution LC, and the type, concentration, etc. of the enzymes in the enzyme reaction solutions LE. Here, the enzymes having the same name but different origins are also classified as different types.

Further, in the enzyme reactor 100 of the first example, the dialysis cup 5 can be mounted on and removed from the reaction vessel 2. Therefore, in a single reaction vessel 2, the plurality of dialysis cups 5 having different types, concentrations, and/or the like of the enzymes in the enzyme reaction solutions LE can be used by placing one another, and an optimum type, concentration, etc. of the enzymes for performing the one-step process can be searched. Therefore, when searching for the optimum type, concentration, etc. of the enzymes, the amount of use of each type of enzyme can be made minute, thus allowing the screening cost to be saved.

### (Second Example)

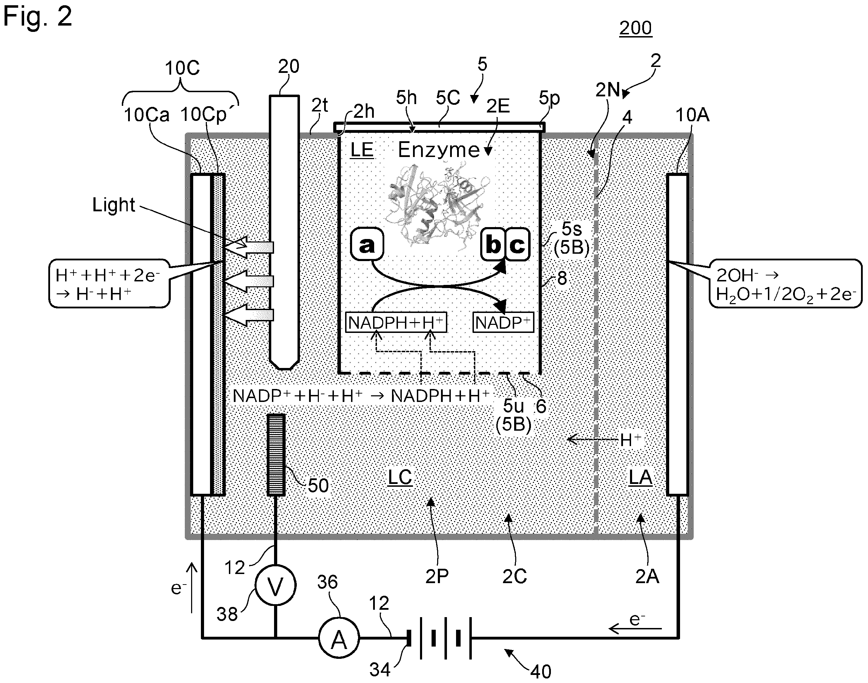

An enzyme reactor of a second example according to the first embodiment will be described mainly in terms of differences from the enzyme reactor of the first example according to the first embodiment. Fig. 2 is a schematic cross-sectional view illustrating the enzyme reactor of the second example according to the first embodiment.

First, a configuration of the enzyme reactor of the second example will be described. As illustrated in Fig. 2, in an enzyme reactor 200 of the second example, a photocatalyst layer 10Cp' including a photocatalyst is formed on the surface of the cathode substrate 10Ca of the cathodic electrode 10C, which is different from the photocatalyst layer 10Cp in the first example.

The enzyme reactor 200 of the second example includes a reference electrode 50 and an external circuit 40 including a wiring 12, an external power supply 34, an ammeter 36, and a voltmeter 38, instead of the external circuit 30 included in the enzyme reactor 100 of the first example. The wiring 12 electrically connects the anodic electrode 10A to the cathodic electrode 10C. The external power supply 34 applies a positive voltage and a negative voltage to the anodic electrode 10A and the cathodic electrode 10C via the wiring 12, respectively. The ammeter 36 is connected to the wiring 12 and measures a current flowing between the anodic electrode 10A and the cathodic electrode 10C. The reference electrode 50 is arranged so as to be immersed in the cathodic electrolytic solution LC in the vicinity of the cathodic electrode 10C in the photocatalytic reaction chamber 2P, and is connected to a negative electrode of the external power supply 34 by the wiring 12. The voltmeter 38 is connected to the reference electrode 50 and the negative electrode of the external power supply 34 by the wiring 12, and measures a potential of the cathodic electrode 10C with respect to a potential of the reference electrode 50.

The configuration of the enzyme reactor 200 of the second example is similar to the enzyme reactor 100 of the first example except as described above.

Next, an example of a method for producing a substance using the enzyme reactor of the second example will be described.

In the example of the method for producing a substance using the enzyme reactor 200 of the second example, as illustrated in Fig. 2, similarly to the example of the method for producing the substance using the enzyme reactor of the first example, first, the light source 20 irradiates the photocatalyst layer 10Cp' of the cathodic electrode 10C with light through the cathodic electrolytic solution LC. At the same time, the positive voltage and the negative voltage are respectively applied to the anodic electrode 10A and the cathodic electrode 10C by the external power supply 34. At this time, the potential of the cathodic electrode 10C with respect to the potential of the reference electrode 50 is set to a predetermined value such that the total voltage of the voltage generated by irradiating the photocatalyst layer 10Cp' with light and the voltage applied by the external power supply 34 is equal to or higher than the voltage required for causing the reduction reaction represented in the above Reaction formula (1-1) to occur. As a result, the excited electrons (e⁻) are generated in the photocatalyst layer 10Cp' of the cathodic electrode 10C, and the reduction reaction represented in the above Reaction formula (1-1) is performed in the vicinity of cathodic electrode 10C in the cathodic electrolytic solution LC, and simultaneously with this reduction reaction, the oxidation reaction represented in the above Reaction formula (1-2) is performed in a vicinity of the anodic electrode 10A in the anodic electrolytic solution LA.

Next, as illustrated in Fig. 2, similarly to the first example, the reduction type electron mediator (NADPH) and the protons (H⁺) are transferred to the enzyme reaction solution LE of the enzymatic reaction chamber 2E by permeating through the semipermeable membrane 6. Next, as illustrated in Fig. 2, similarly to the first example, in the enzyme reaction solution LE, the biochemical reaction (enzymatic reaction) represented in the above Reaction formula (1-3) occurs. This produces the mevalonate ((R)-mevalonate) and the coenzyme A (CoA) as the substance [b] and the substance [c], respectively, while simultaneously producing the oxidation type electron mediator (NADP⁺).

Next, although not illustrated, similarly to the first example, the dialysis cup 5 is removed from the mounting port 2h of the reaction vessel 2, subsequently the cover portion 5C of the dialysis cup 5 is opened, and the substance [b] (mevalonate) and the substance [c] (coenzyme A) are recovered from the enzyme reaction solution LE inside the dialysis cup 5.

As described above, the enzyme reactor 200 of the second example includes the reference electrode 50 and the external circuit 40 including the wiring 12, the external power supply 34, the ammeter 36, and the voltmeter 38, unlike the enzyme reactor 100 of the first example. Therefore, when only the voltage generated by irradiating the photocatalyst layer 10Cp' with light is insufficient as the voltage required for causing the reduction reaction represented in the above-described Reaction formula (1-1) to occur, compensating for the insufficiency by applying a voltage by the external power supply 34 allows producing the high value-added substance [b] from the raw material substrate [a] with the process in which the reduction reaction and the biochemical reaction are linked. At this time, by setting the potential of the cathodic electrode 10C with respect to the potential of the reference electrode 50 to a predetermined value, the total voltage of the voltage generated by irradiating the photocatalyst layer 10Cp' with light and the voltage applied by the external power supply 34 can be set to be equal to or higher than the voltage required for causing the reduction reaction to occur.

In the enzyme reactor 200 of the second example, when the light source 20 irradiates the photocatalyst layer 10Cp' of the cathodic electrode 10C with light through the cathodic electrolytic solution LC without applying the voltage by the external power supply 34, measuring the potential of the cathodic electrode 10C with respect to the potential of the reference electrode 50 with the voltmeter 38 allows determining whether the voltage generated by irradiating the photocatalyst layer 10Cp' with light is sufficient as the voltage required for causing the reduction reaction to occur or not from the measured value of the potential.

### (Third Example)

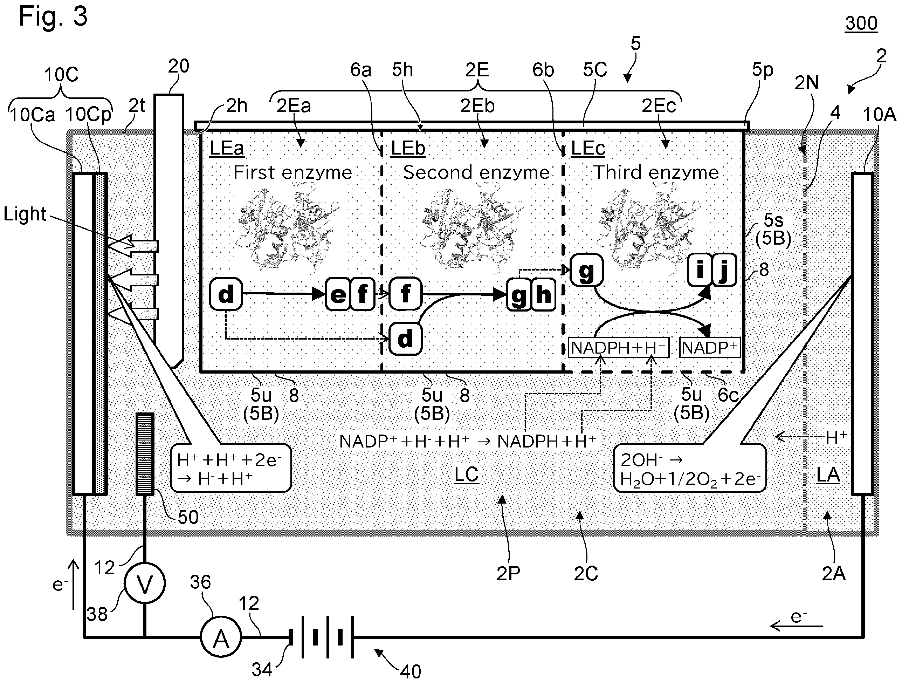

An enzyme reactor of a third example according to the first embodiment will be described mainly in terms of differences from the enzyme reactor of the first example according to the first embodiment. Fig. 3 is a schematic cross-sectional view illustrating the enzyme reactor of the third example according to the first embodiment.

First, a configuration of the enzyme reactor of the third example will be described.

As illustrated in Fig. 3, in an enzyme reactor 300 of the third example, a reaction vessel 2 and a dialysis cup 5 are laterally larger than those of the enzyme reactor 100 of the first example, and a main body portion 5B of the dialysis cup 5 has a quadrangular prism shape. Unlike the enzyme reactor 100 of the first example, the enzyme reactor 300 of the third example further includes a first semipermeable membrane 6a and a second semipermeable membrane 6b that partition the enzymatic reaction chamber 2E inside the dialysis cup 5 into a first enzymatic reaction chamber 2Ea, a second enzymatic reaction chamber 2Eb, and a third enzymatic reaction chamber 2Ec in a direction parallel to a bottom surface of the main body portion 5B. The first enzymatic reaction chamber 2Ea and the second enzymatic reaction chamber 2Eb are partitioned by the first semipermeable membrane 6a. The second enzymatic reaction chamber 2Eb and the third enzymatic reaction chamber 2Ec are partitioned by the second semipermeable membrane 6b. In the bottom portion 5u of the main body portion 5B of the dialysis cup 5, a portion corresponding to the bottom portion of the third enzymatic reaction chamber 2Ec is formed of a third semipermeable membrane 6c, and the other portion is formed of the non-permeable membrane 8. A side portion 5s of the main body portion 5B is made of the non-permeable membrane 8. In a cathode chamber 2C, a photocatalytic reaction chamber 2P and the first enzymatic reaction chamber 2Ea are partitioned by the bottom portion 5u and the non-permeable membrane 8 of the side portion 5s of the main body portion 5B of the dialysis cup 5. A photocatalytic reaction chamber 2P and the second enzymatic reaction chamber 2Eb are partitioned by the non-permeable membrane 8 of the bottom portion 5u of the main body portion 5B of the dialysis cup 5. Also, the photocatalytic reaction chamber 2P and the third enzymatic reaction chamber 2Ec are partitioned by the third semipermeable membrane 6c of the bottom portion 5u and the non-permeable membrane 8 of the side portion 5s of the main body portion 5B of the dialysis cup 5.

Unlike the enzyme reactor 100 of the first example, the enzyme reactor 300 of the third example includes a first enzyme reaction solution LEa housed in the first enzymatic reaction chamber 2Ea, a second enzyme reaction solution LEb housed in the second enzymatic reaction chamber 2Eb, and a third enzyme reaction solution LEc housed in the third enzymatic reaction chamber 2Ec, instead of the enzyme reaction solution LE housed in the enzymatic reaction chamber 2E.

The anodic electrolytic solution LA is a predetermined electrolytic solution in which a predetermined electrolyte is dissolved in water. The cathodic electrolytic solution LC is obtained by dissolving the oxidation type electron mediator (NADP⁺) in the common (same) electrolytic solution with the anodic electrolytic solution LA. The first enzyme reaction solution LEa is obtained by adding Acetyl-CoA acetyltransferase (ACAT) as a first enzyme and Acetyl-CoA as a raw material substrate [d] to the common electrolytic solution with the anodic electrolytic solution LA. The second enzyme reaction solution LEb is obtained by adding Hydroxymethylglutaryl-CoA synthase (HMGS) as a second enzyme to the common electrolytic solution with the anodic electrolytic solution LA. The third enzyme reaction solution LEc is obtained by adding 3-hydroxy-3-methylglutaryl coenzyme A reductase (HMGR) as a third enzyme to the common electrolytic solution with the anodic electrolytic solution LA. As the common electrolytic solution, specifically, for example, a mixed aqueous solution (buffer solution) of a weak acid or a weak base and a salt thereof, pH of which is adjusted within a range near the optimum pH of the first to third enzymes, and the temperature of which is adjusted within the range near the optimum temperature of the first to third enzymes is employed. Examples of the optimum electrolytic solution as the above-described mixed aqueous solution (buffer solution) in which the pH is adjusted within the range near the optimum pH and the temperature is adjusted within the range near the optimum temperature include a mixed aqueous solution of, for example, 1 mM of MgCl₂, 2 mM of KCl, and 50 mM of TrisHCl (pH 7.2).

The first enzyme reaction solution LEa and the second enzyme reaction solution LEb are in contact with one another via the first semipermeable membrane 6a. The second enzyme reaction solution LEb and the third enzyme reaction solution LEc are in contact with one another via the second semipermeable membrane 6b. The third enzyme reaction solution LEc and the cathodic electrolytic solution LC are in contact with one another via the third semipermeable membrane 6c. The first semipermeable membrane 6a is a membrane that allows the raw material substrate [d] and a substance [f] to be described later to permeate through the membrane, and does not allow the first to third enzymes to permeate through the membrane. The second semipermeable membrane 6b is a membrane that allows a substance [g] to be described later to permeate through the membrane and does not allow the first to third enzymes to permeate through the membrane. The third semipermeable membrane is a membrane that allows the oxidation type electron mediator (NADP⁺), the reduction type electron mediator (NADPH) that is the oxidation type electron mediator (NADP⁺) having undergone the reduction, and the protons (H⁺) to permeate through the membrane and does not allow the first to third enzymes to permeate through the membrane. The non-permeable membrane 8 is a membrane that does not allow any component of the cathodic electrolytic solution LC and the first to third enzyme reaction solutions to permeate through the membrane.

The enzyme reactor 300 of the third example includes the reference electrode 50 and the external circuit 40 including the wiring 12, the external power supply 34, the ammeter 36, and the voltmeter 38, instead of the external circuit 30 included in the enzyme reactor 100 of the first example. The configurations of the wiring 12, the external power supply 34, the ammeter 36, and the voltmeter 38 of the external circuit 40, and the reference electrode 50 are similar to those of the enzyme reactor 200 of the second example.

The configuration of the enzyme reactor 300 of the third example is similar to the enzyme reactor 100 of the first example except as described above.

Next, an example of a method for producing a substance using the enzyme reactor of the third example will be described.

In the example of the method for producing the substance using the enzyme reactor 300 of the third example, as illustrated in Fig. 3, first, similarly to the example of the method for producing the substance using the enzyme reactor of the first example, the light source 20 irradiates the photocatalyst layer 10Cp of the cathodic electrode 10C with light through the cathodic electrolytic solution LC. At the same time, the positive voltage and the negative voltage are respectively applied to the anodic electrode 10A and the cathodic electrode 10C by the external power supply 34. At this time, the potential of the cathodic electrode 10C with respect to the potential of the reference electrode 50 is set to a predetermined value such that the total voltage of the voltage generated by irradiating the photocatalyst layer 10Cp with light and the voltage applied by the external power supply 34 is equal to or higher than the voltage required for causing the reduction reaction represented in the above Reaction formula (1-1) to occur. As a result, the excited electrons (e⁻) are generated in the photocatalyst layer 10Cp of the cathodic electrode 10C, and the reduction reaction represented in the above Reaction formula (1-1) is performed in the vicinity of the cathodic electrode 10C in the cathodic electrolytic solution LC, and simultaneously with this reduction reaction, the oxidation reaction represented in the above Reaction formula (1-2) is performed in the vicinity of the anodic electrode 10A in the anodic electrolytic solution LA.

On the other hand, in the enzyme reactor 300 of the third example, the biochemical reaction (enzymatic reaction) represented in Reaction formula (1-4) below occurs in the first enzyme reaction solution LEa, as illustrated in Fig. 3, prior to and in parallel with the reduction reaction and the oxidation reaction described above. In this biochemical reaction, the first enzyme (ACAT) functions as a reductase to reduce the raw material substrate [d] (Acetyl-CoA). This produces coenzyme A (CoA) and Acetoacetyl-CoA as substance [e] and substance [f], respectively.

2Acetyl-CoA → CoA + Acetoacetyl-CoA (1-4)

Then, as illustrated in Fig. 3, the raw material substrate [d] (Acetyl-CoA) and the substance [f] (Acetoacetyl-CoA) in the first enzyme reaction solution LEa are transferred to the second enzyme reaction solution LEb by permeating through the first semipermeable membrane 6a. Consequently, in the second enzyme reaction solution LEb, the biochemical reaction (enzymatic reaction) represented in Reaction formula (1-5) below occurs. In this biochemical reaction, the second enzyme (HMGS) functions as a reductase to reduce the raw material substrate [d] (Acetyl-CoA) and the substance [f] (Acetoacetyl-CoA). This produces 3-hydroxy-3-methylglutaryl-CoA (HMG-CoA) and coenzyme A (CoA) as a substance [g] and a substance [h], respectively.

Acetyl-CoA + H₂O + Acetoacetyl-CoA → HMG-CoA + CoA (1-5)

Subsequently, as illustrated in Fig. 3, the substance [g] (HMG-CoA) in the second enzyme reaction solution LEb is transferred to the third enzyme reaction solution LEc by permeating through the second semipermeable membrane 6b. Further, the reduction type electron mediator (NADPH) generated by the reduction reaction represented in the above Reaction formula (1-1) and the protons (H⁺) remaining in the reduction reaction are transferred to the third enzyme reaction solution LEc by permeating through the third semipermeable membrane 6c. Consequently, in the third enzyme reaction solution LEc, the biochemical reaction (enzymatic reaction) represented in Reaction formula (1-6) below occurs. In this biochemical reaction, the third enzyme (HMGR) functions as a reductase and the reduction type electron mediator (NADPH) functions as an energy-imparting coenzyme to reduce the substance [g] (HMG-CoA). This produces mevalonate ((R)-mevalonate) and coenzyme A (CoA) as the substance [i] and the substance [j], respectively. At the same time, the reduction type electron mediator (NADPH) is oxidized to produce the oxidation type electron mediator (NADP⁺).

HMG-CoA + 2NADPH + 2H⁺ → (R)-mevalonate + CoA + 2NADP⁺ (1-6)

Next, although not illustrated, after the dialysis cup 5 is removed from the mounting port 2h of the reaction vessel 2, the cover portion 5C of the dialysis cup 5 is opened, and the substance [i] (mevalonate ((R)-mevalonate)) and the substance [j] (coenzyme A (CoA)) are recovered from the third enzyme reaction solution LEc inside the dialysis cup 5.

As described above, in the enzyme reactor 300 of the third example, the enzymatic reaction chamber 2E is partitioned into the first enzymatic reaction chamber 2Ea, the second enzymatic reaction chamber 2Eb, and the third enzymatic reaction chamber 2Ec in the reaction vessel 2 as a single reaction cell. The first enzymatic reaction chamber 2Ea and the second enzymatic reaction chamber 2Eb are partitioned by the first semipermeable membrane 6a that allows the raw material substrate [d] and the substance [f] to permeate through the membrane, and does not allow the first to third enzymes to permeate through the membrane. The second enzymatic reaction chamber 2Eb and the third enzymatic reaction chamber 2Ec are partitioned by the second semipermeable membrane 6b that allows the substance [g] to permeate through the membrane and does not allow the first to third enzymes to permeate through the membrane. The third enzymatic reaction chamber 2Ec and the photocatalytic reaction chamber 2P are partitioned by the third semipermeable membrane 6c that allows the oxidation type electron mediator (NADP⁺), the reduction type electron mediator (NADPH), and the protons (H⁺) to permeate through the membrane and does not allow the first to third enzymes to permeate through the membrane. The photocatalytic reaction chamber 2P houses the cathodic electrolytic solution LC in which the oxidation type electron mediator (NADP⁺) is dissolved in the common electrolytic solution, the first enzymatic reaction chamber 2Ea houses the first enzyme reaction solution LEa in which the first enzyme (ACAT) and the raw material substrate [d] (Acetyl-CoA) are added to the common electrolytic solution, the second enzymatic reaction chamber 2Eb houses the second enzyme reaction solution LEb in which the second enzyme (HMGS) is added to the common electrolytic solution, and the third enzymatic reaction chamber 2Ec houses the third enzyme reaction solution LEc in which the third enzyme (HMGR) is added to the common electrolytic solution. For this reason, the reduction reaction in which the oxidation type electron mediator (NADP⁺) is reduced by the reduction action of the photocatalyst in the cathodic electrolytic solution LC to produce the reduction type electron mediator (NADPH); and a three-step biochemical reaction (enzymatic reaction) for producing the substance [i] and the substance [j] from the raw material substrate [d] by causing the first to third enzymes to function in the first to third enzyme reaction solutions and using the reduction type electron mediator as the coenzyme are allowed to be linked and performed. That is, the enzymatic reaction chamber is partitioned and multi-layered into the plurality of enzymatic reaction chambers by the plurality of semipermeable membranes, thus allowing a multi-stage enzymatic reaction.

Furthermore, in the enzyme reactor 300 of the third example, the biochemical reaction by the first enzyme, the biochemical reaction by the second enzyme, and the biochemical reaction by the third enzyme can be individually performed in the first to third enzymatic reaction chambers. This makes it easy to monitor the individual progress of these biochemical reactions. Therefore, the replacement and addition of the old and new enzymes of the first to third enzymes in the first to third enzymatic reaction chambers can be easily performed based on the monitoring.

Hereinafter, the configuration of the enzyme reactor according to the first embodiment will be described in detail.

### 1. Semipermeable Membrane

The semipermeable membrane is a membrane that partitions the cathode chamber into the photocatalytic reaction chamber and the enzymatic reaction chamber, allows the reduced electron mediator (the substance which is obtained by reducing the electron mediator) to permeate through the membrane, and does not allow the enzyme to permeate through the membrane.

The molecular weight cutoff (MWCO) of the semipermeable membrane is not particularly limited as long as the semipermeable membrane has an amount that allows the reduced electron mediator (the substance which is obtained by reducing the electron mediator) to permeate through the membrane and does not allow the enzymes to permeate through the membrane. For example, it is preferably within a range of 1000 Da or more and 10000 Da or less. This is because the semipermeable membrane can preferably be a membrane that allows the electron mediator to permeate through the membrane and does not allow the enzymes to permeate through the membrane.

The material of the semipermeable membrane is not particularly limited, and for example, a porous polymer membrane can be employed. As a polymer material used for the porous polymer membrane, polytetrafluoroethylene is particularly preferable because of its high biocompatibility.

### 2. Anodic electrolytic solution, Cathodic electrolytic solution, and Enzyme Reaction Solution

The anodic electrolytic solution is an electrolytic solution in which an electrolyte is dissolved in a solvent. The cathodic electrolytic solution is not particularly limited as long as the cathodic electrolytic solution is obtained by dissolving the electron mediator in the electrolytic solution, but is usually a solution in which the electron mediator is dissolved in the common (same) electrolytic solution with the anodic electrolytic solution. The enzyme reaction solution is not particularly limited as long as the enzyme reaction solution is obtained by adding the enzymes and the raw material substrate to a liquid such as water. For example, the enzyme reaction solution is obtained by adding the enzymes and the raw material substrate to the common electrolytic solution with the anodic electrolytic solution.

The common electrolytic solution employed for the anodic electrolytic solution, the cathodic electrolytic solution, and the enzyme reaction solution is not particularly limited as long as the common electrolytic solution is an electrolytic solution that can link and perform the target reduction reaction in the cathodic electrolytic solution and the target enzyme reaction in the enzyme reaction solution. However, the common electrolytic solution is preferably, for example, an electrolytic solution in which an electrolyte is dissolved in a solvent such as water, and among them, a mixed aqueous solution (buffer solution) of a weak acid or a weak base and a salt thereof, pH of which is adjusted within a range near the optimum pH of the enzyme, and the temperature is adjusted within a range near the optimum temperature of the enzyme.

The electron mediator contained in the cathodic electrolytic solution is not particularly limited as long as the electron mediator can be reduced by a voltage applied to the cathodic electrode and the reduced electron mediator can be used as a coenzyme in the target enzymatic reaction in the enzyme reaction solution, and examples thereof include nicotinamide adenine dinucleotide phosphate (NADP⁺), nicotinamide adenine dinucleotide (NAD⁺), and flavin adenine dinucleotide (FAD).

The concentration [ppm] of the electron mediator in the cathodic electrolytic solution is not particularly limited as long as the target reduction reaction in the cathodic electrolytic solution and the target enzymatic reaction in the enzyme reaction solution can be linked and performed. The concentration may be appropriately selected depending on the conditions including the type, etc. of the electrolytic solution, the type, etc. of the reduction reaction, the type, etc. of the electron mediator, the type, etc. of the enzymatic reaction, the type, concentration, etc. of the enzymes, the type, concentration, etc. of the substrate, and the like.

The enzyme contained in the enzyme reaction solution is not particularly limited as long as the enzyme is a molecule which functions as a catalyst for the target enzyme reaction in the enzyme reaction solution, and the enzyme may be appropriately selected according to the target enzyme reaction in the enzyme reaction solution. Examples of the enzyme include an oxidoreductase, a transferase, a hydrolase, a lyase, an isomerase, and a ligase (synthase), and among them, for example, an enzyme using the electron mediator as the coenzyme contained in the cathodic electrolytic solution is preferred.

The concentration [U/mL] of the enzyme in the enzyme reaction solution is not particularly limited as long as the target reduction reaction in the cathodic electrolytic solution and the target enzyme reaction in the enzyme reaction solution are allowed to be linked and performed. The concentration may be appropriately selected according to the condition including the type, etc. of the electrolytic solution, the type, etc. of the reduction reaction, the type, concentration, etc. of the electron mediator, the type, etc. of the enzyme reaction, the type, etc. of the enzyme, and the type, concentration, etc. of the substrate, and the like.

The raw material substrate contained in the enzyme reaction solution is not particularly limited as long as the raw material substrate is a chemical species that serves as a raw material for the substance generated by the target enzyme reaction in the enzyme reaction solution and the raw material substrate is a substance that causes the enzyme reaction of the substance to be catalyzed by the enzyme, and the raw material substrate may be appropriately selected according to the target enzyme reaction in the enzyme reaction solution.

The concentration [mol/mL] of the raw material substrate in the enzyme reaction solution is not particularly limited as long as the target reduction reaction in the cathodic electrolytic solution and the target enzyme reaction in the enzyme reaction solution are allowed to be linked and performed. The concentration may be appropriately selected according to the conditions including the type, etc. of the electrolytic solution, the type, etc. of the reduction reaction, the type, concentration, etc. of the electron mediator, the type, etc. of the enzyme reactions, the type, concentration, etc. of the enzymes, the type, etc. of the substrate.

### 3. Anodic Electrode and Cathodic Electrode

The anodic electrode is arranged in the anode chamber so as to be immersed in the anodic electrolytic solution. The material of the anodic electrode is not particularly limited as long as the material has a conductivity and does not deteriorate in the oxidation reaction occurring in the vicinity of the anodic electrode. Examples thereof include a metal substrate, a glass substrate, and the like, and among them, a metal substrate and the like made of platinum, gold, silver, copper, titanium, and the like are preferred.

The cathodic electrode is arranged in the photocatalytic reaction chamber so as to be immersed in the cathodic electrolytic solution, and has a photocatalyst layer containing a photocatalyst on a surface thereof. The cathodic electrode preferably includes, for example, a cathode substrate and a photocatalyst layer formed on a surface of the cathode substrate. The material of the cathode substrate is not particularly limited as long as the material has a conductivity and does not deteriorate in the reduction reaction occurring in the vicinity of the cathodic electrode. Examples thereof include a metal substrate, a glass substrate, and the like, and among them, a metal substrate and the like made of platinum, gold, silver, copper, titanium, and the like are preferred.

The photocatalyst layer includes a photocatalyst, thus generates the excited electrons (e⁻) by being irradiated with light, and exhibits the reducing action. The photocatalyst layer is not particularly limited as long as the photocatalyst layer contains a photocatalyst. However, the photocatalyst layer containing a co-catalyst supported on the photocatalyst in addition to the photocatalyst is preferred. Examples of the material of the photocatalyst include metal oxides, such as transition-metal oxides, and among them, BiVO₄, SrTiO₃, NaTaO₃, WO₃, Cu oxides, and the like are preferred. Preferable examples of the material of the co-catalyst includes CoO, NiO, FeO, Y₂O₃, Rh, Ru, Ir, and CoPₓ.

### 4. Ion Exchange Membrane

The ion exchange membrane partitions the inside of the reaction vessel into the anode chamber and the cathode chamber, and allows only predetermined ions contained in the electrolytic solution to permeate through the membrane. The ion exchange membrane is not particularly limited, and for example, the proton exchange membrane and the like that allows only the protons (H⁺) to permeate through the membrane and does not allow other substances to permeate through the membrane is preferred. The material of the proton exchange membrane is not particularly limited and may be appropriately selected according to the various conditions. Examples of the material include Nafion (registered trademark) and the like.

### 5. Enzyme Reactor

The enzyme reactor according to the first embodiment includes: the reaction vessel; the ion exchange membrane; the semipermeable membrane; the anodic electrolytic solution; the cathodic electrolytic solution; the enzyme reaction solution; the anodic electrode; and the cathodic electrode. The cathodic electrolytic solution and the enzyme reaction solution are in contact via the semipermeable membrane.

### (1) Other configurations

The enzyme reactor according to the first embodiment is not particularly limited as long as the enzyme reactor is as described above, but usually further includes a light source capable of irradiating the photocatalyst layers of the cathodic electrode with light, similarly to the first example to the third example. The light source is not particularly limited as long as the light source generates a voltage by irradiating the photocatalyst layer with light, and examples thereof include halogen lamps, LED lamps, and natural lights.

Preferably, similarly to the enzyme reactors of the second example and the third example, the enzyme reactor preferably further includes the reference electrode arranged so as to be immersed in the cathodic electrolytic solution in the vicinity of the cathodic electrode in the photocatalytic reaction chamber and is capable of measuring the potential of the cathodic electrode with respect to the potential of the reference electrode. This is because it is possible to determine whether or not the voltage generated by irradiating the photocatalyst layer with light is sufficient as the voltage required for causing the reduction reaction to occur.

Similarly to the enzyme reactors of the second example and the third example, the enzyme reactor preferably further includes the external power supply that applies the positive voltage and the negative voltage to the anodic electrode and the cathodic electrode, respectively. This is because, when the voltage generated by irradiating the photocatalyst layer with light alone is insufficient as the voltage required for causing the reduction reaction to occur, compensating for the insufficiency by applying the voltages by the external power supply allows the process in which the reduction reaction and the enzyme reaction are linked to be performed.

Preferably, similarly to the enzyme reactor of the first example to the third example, the enzyme reactor includes the dialysis cup and the mounting port that allows mounting and removing of the dialysis cup at a portion of the cathode chamber side in the upper portion of the reaction vessel. A part of the dialysis cup is preferred to be the semipermeable membrane that partitions the cathode chamber into the photocatalytic reaction chamber and the enzymatic reaction chamber. This is because, in the single reaction vessel, the plurality of dialysis cups having different types, concentrations, and/or the like of the enzymes in the enzyme reaction solutions can be used by replacing one another, and the optimum type, concentration, and the like of the enzymes for performing the process in which the reduction reaction and the enzyme reaction are linked can be searched.

Preferably, similarly to the enzyme reactor of the third example, in the enzyme reactor, the enzymatic reaction chamber is partitioned and multi-layered into the plurality of enzymatic reaction chambers by the one or more semipermeable membranes, the plurality of types of enzyme reaction solutions housed in the respective plurality of enzymatic reaction chambers contain the enzymes, and at least one of the plurality of types of enzyme reaction solutions further contains the raw material substrate. This is because, the multi-stage enzymatic reaction is allowed.

Among the above-described devices, the enzyme reactor is preferred to be a device in which the cathode solution housed in the photocatalytic reaction chamber and the plurality of types of the enzyme reaction solutions housed in the respective plurality of the enzymatic reaction chambers are in contact with one another via the semipermeable membranes according to the designs of the reaction pathway. This is because the electron mediator intercommunicates through the plurality of transfer paths between the cathode solution and the plurality of types of enzyme reaction solutions, which allows the multi-stage enzymatic reaction with the electron mediator functioning as a coenzyme and allows producing the higher value-added substance.

Among the above-described devices in which the enzymatic reaction chambers are partitioned and multi-layered into the plurality of enzymatic reaction chambers, the enzyme reactor is preferred to be a device in which the reaction pathway is configured to be a desired pathway by especially configuring the permeabilities of the raw material substrate and the respective plurality of substances produced by the enzymatic reactions at the plurality of enzymatic reaction chambers for the semipermeable membrane between the photocatalytic reaction chamber and the enzymatic reaction chambers and the one or more semipermeable membrane between the plurality of enzymatic reaction chambers. This is because the complicated reaction pathway is allowed.

Among the above-described devices in which the enzymatic reaction chambers are partitioned and multi-layered into the plurality of enzymatic reaction chambers, the enzyme reactor is preferred to be a device in which the semipermeable membrane between the photocatalytic reaction chamber and the enzymatic reaction chamber and the one or more semipermeable membrane between the plurality of the enzymatic reaction chamber have different amounts (for example, different amounts within the range of 1000 Da or more and 10000 Da or less) of molecular weight cutoff (MWCO).

### (2) Application

The enzyme reactor according to the first embodiment is used in the method for producing the substance from the raw material substrate by irradiating the photocatalyst layer of the cathodic electrode with light to allow the reduction reaction (sometimes referred to as "target reduction reaction in the cathodic electrolytic solution" in the first embodiment) in which the electron mediator is reduced in the cathodic electrolytic solution and the enzyme reaction (sometimes referred to as "target enzyme reaction in the enzyme reaction solution" in the first embodiment) in which the enzyme is caused to function in the enzyme reaction solution and the electron mediator reduced by the reduction reaction is used as the coenzyme to produce the substance from the raw material substrate, to be linked and performed. The use of the enzyme reactor is not particularly limited as long as the use is the method for producing the substance, but it may be a method for producing a substance from a raw material substrate by, for example, performing a reduction reaction and a biochemical reaction (enzymatic reaction) in a linked manner.

### B. Second Embodiment

An enzyme reactor according to the second embodiment includes a reaction vessel; an ion exchange membrane that partitions an inside of the reaction vessel into an anode chamber and a cathode chamber; a semipermeable membrane that partitions the cathode chamber into a photocatalytic reaction chamber and an enzymatic reaction chamber; an anodic electrolytic solution housed in the anode chamber; a cathodic electrolytic solution housed in the photocatalytic reaction chamber; an enzyme reaction solution housed in the enzymatic reaction chamber; an anodic electrode disposed in the anode chamber so as to be immersed in the anodic electrolytic solution; and a cathodic electrode disposed in the photocatalytic reaction chamber so as to be immersed in the cathodic electrolytic solution. The cathodic electrode has a surface on which a photocatalyst layer containing a photocatalyst is disposed. The cathodic electrolytic solution contains carbon dioxide. The enzyme reaction solution contains an enzyme. The cathodic electrolytic solution and the enzyme reaction solution are in contact with one another via the semipermeable membrane. The semipermeable membrane allows the reduced carbon dioxide to permeate through the semipermeable membrane and does not allow the enzyme to permeate through the semipermeable membrane.

### (Fourth Example)

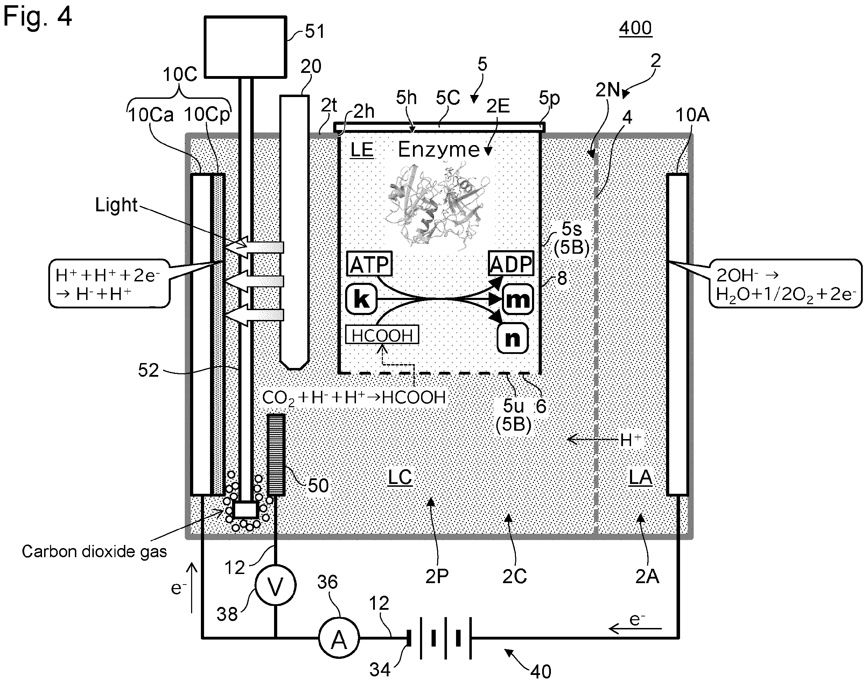

The enzyme reactor of the fourth example according to the second embodiment will be mainly described in terms of differences from the enzyme reactor of the first example of the first embodiment. Fig. 4 is a schematic cross-sectional view illustrating the enzyme reactor of the fourth example according to the second embodiment.

First, a configuration of the enzyme reactor of the fourth example will be described. As illustrated in Fig. 4, in an enzyme reactor 400 of the fourth example, an anodic electrolytic solution LA is the predetermined electrolytic solution in which the predetermined electrolyte is dissolved in water. A cathodic electrolytic solution LC is the solution in which the carbon dioxide (CO₂) is dissolved in the common (same) electrolytic solution with the anodic electrolytic solution LA. An enzyme reaction solution LE is obtained by adding formate-tetrahydrofolate ligase (FTL) as an enzyme, adding Tetrahydrofolic acid (THF) as a raw material substrate [k], and adding adenosine triphosphate (ATP) to the common electrolytic solution with the anodic electrolytic solution LA. As the common electrolytic solution, for example, a mixed aqueous solution (buffer solution) of a weak acid or a weak base and a salt thereof, pH of which is adjusted within a range near the optimum pH of the enzyme (FTL), and the temperature is adjusted within a range near the optimum temperature of the enzyme (FTL) is used.

The anodic electrolytic solution LA and the cathodic electrolytic solution LC are in contact with one another via the proton exchange membrane 4. The cathodic electrolytic solution LC and the enzyme reaction solution LE are in contact with one another via the semipermeable membrane 6. The semipermeable membrane 6 is a membrane that allows formic acid (HCOOH) which is obtained by reducing carbon dioxide (CO₂) to permeate through the membrane and does not allow enzyme (FTL) to permeate through the membrane. It is preferred that the semipermeable membrane 6 be a membrane that allows formic acid (HCOOH) to permeate through the membrane and does not allow enzyme (FTL), the raw material substrate [k], ATP, a substance [m] and a substance [n] to be described later, and ADP to be described later to permeate through the membrane. This is because formation of the substance [m] and the substance [n] is likely to occur, and a recovery of the substance [m] and the substance [n] is facilitated. However, when the molecular weights of the raw material substrate [k], ATP, the substance [m] and the substance [n] to be described later, and ADP to be described later are not significantly higher than that of formic acid (HCOOH) and an appropriate semipermeable membrane cannot be selected, the raw material substrate [k], the ATP, the substance [m] and the substance [n] to be described later, and the ADP to be described later become diffused into both the cathodic electrolytic solution LC and the enzyme reaction solution LE. However, even in this state, the reduction reaction and the biochemical reaction (enzymatic reaction) can be linked and performed as described later.

The enzyme reactor 400 of the fourth example includes the reference electrode 50 and the external circuit 40 including the wiring 12, the external power supply 34, the ammeter 36, and the voltmeter 38, instead of the external circuit 30 included in the enzyme reactor 100 of the first example according to the first embodiment. The configurations of the wiring 12, the external power supply 34, the ammeter 36, and the voltmeter 38 of the external circuit 40 and the reference electrode 50 are similar to those of the enzyme reactor 200 of the second example according to the first embodiment.

Unlike the enzyme reactor 100 of the first example according to the first embodiment, the enzyme reactor 400 of the fourth example includes a carbon dioxide feeder 51 and a gas introduction pipe 52. One end of the gas introduction pipe 52 is connected to the carbon dioxide feeder 51, and the other end of the gas introduction pipe 52 is immersed in the cathodic electrolytic solution LC. The carbon dioxide feeder 51 feeds carbon dioxide (CO₂) gas to the cathodic electrolytic solution LC through the gas introduction pipe 52. As a result, bubbles of the carbon dioxide gas are generated in the cathodic electrolytic solution LC, and the carbon dioxide is dissolved in the cathodic electrolytic solution LC.

A configuration of the enzyme reactor 400 of the fourth example is similar to that of the enzyme reactor 100 of the first example according to the first embodiment, except as described above.

Next, an example of a method for producing a substance using the enzyme reactor 400 of the fourth example will be described. In the example of the method for producing a substance using the enzyme reactor 400 of the fourth example, as illustrated in Fig. 4, first, carbon dioxide (CO₂) gas is fed into the cathodic electrolytic solution LC through the gas introduction pipe 52 by the carbon dioxide feeder 51. While the carbon dioxide is thus dissolved in the cathodic electrolytic solution LC, the light source 20 irradiates the photocatalyst layer 10Cp of the cathodic electrode 10C with light through the cathodic electrolytic solution LC similarly to the example of the method for producing the substance using the enzyme reactor of the first example. At the same time, the positive voltage and the negative voltage are respectively applied to the anodic electrode 10A and the cathodic electrode 10C by the external power supply 34. At this time, the potential of the cathodic electrode 10C with respect to the potential of the reference electrode 50 is set to a predetermined value such that the total voltage of the voltage generated by irradiating the photocatalyst layer 10Cp with light and the voltage applied by the external power supply 34 is equal to or higher than the voltage required for causing the reduction reaction represented in Reaction formula (2-1) below to occur. As a result, the excited electrons (e⁻) are generated on the photocatalyst layer 10Cp of the cathodic electrode 10C, and the reduction reaction represented in Reaction formula (2-1) below is performed in the vicinity of the cathodic electrode 10C in the cathodic electrolytic solution LC. In this reduction reaction, first, the protons (H⁺) contained in the cathodic electrolytic solution LC are reduced by the excited electrons (e⁻), hydride ions (H⁻) are generated, and then, carbon dioxide (CO₂) is reduced by the hydride ions (H⁻) to produce formic acid (HCOOH). At the same time as the reduction reaction, an oxidizing reaction represented in Reaction formula (2-2) below occurs in the vicinity of the anodic electrode 10A in the anodic electrolytic solution LA. In this oxidation reaction, hydroxide ions (OH⁻) lose the electrons, and water (H₂O) and oxygen (O₂) are formed.

CO₂ + 2H⁺ + 2e⁻ → CO₂ + H⁻ + H⁺ → HCOOH (2-1)

2OH⁻ → H₂O + 1/2O₂ + 2e (2-2)

Next, as illustrated in Fig. 4, formic acid (HCOOH) produced by the above-described reduction is transferred to the enzyme reaction solution LE of the enzymatic reaction chamber 2E by permeating through the semipermeable membrane 6.

Next, as described above, formic acid (HCOOH) is transferred to the enzyme reaction solution LE, and as illustrated in Fig. 4, the biochemical reaction (enzymatic reaction) represented in Reaction formula (2-3) below occurs in the enzyme reaction solution LE. In this biochemical reaction, the enzyme (FTL) functions as a synthase, and the adenosine triphosphate (ATP) gives energy to synthesize 10-Formyl-THF (10-Formyl-tetrahydrofolate) as the substance [m] from the raw material substrate [k] (THF) and formic acid (HCOOH). At the same time, orthophosphate (H₃PO₄) is formed as the substance [n], and adenosine diphosphate (ADP) is produced.

THF + HCOOH + ATP → 10-Formyl-THF + H₃PO₄ + ADP (2-3)

Next, although not illustrated, after the dialysis cup 5 is removed from the mounting port 2h of the reaction vessel 2, the cover portion 5C of the dialysis cup 5 is opened, and the substance [m] (10-Formyl-THF), the substance [n] (orthophosphate), and adenosine diphosphate (ADP) are recovered from the enzyme reaction solution LE inside the dialysis cup 5.

As described above, in the enzyme reactor 400 of the fourth example, the cathode chamber 2C is partitioned into the photocatalytic reaction chamber 2P and the enzymatic reaction chamber 2E by the semipermeable membrane 6, which allows formic acid (HCOOH) to permeate through the membrane and does not allow enzyme (FTL) to permeate through the membrane. The photocatalytic reaction chamber 2P houses the cathodic electrolytic solution LC in which carbon dioxide (CO₂) is dissolved in the common electrolytic solution, and the enzymatic reaction chamber 2E houses the enzyme reaction solution LE in which enzyme (FTL), the raw material substrate [k] (THF), and adenosine triphosphate (ATP) are added to the common electrolytic solution. Therefore, irradiating the photocatalyst layer 10Cp of the cathodic electrode 10C with light and applying a voltage with the external power supply 34 at the same time allows a reduction reaction in which carbon dioxide (CO₂) is reduced to produce formic acid (HCOOH) by the reduction action of the photocatalyst and the like in the cathodic electrolytic solution LC and a biochemical reaction (enzymatic reaction) in which the substance [m] (10-Formyl-tetrahydrofolate) is generated using the raw material substrate [k] (THF) and formic acid (HCOOH) in the enzyme reaction solution LE to be linked and performed. Therefore, the use of the energy of the voltage applied by the external power supply together with the energy generated by irradiating the photocatalyst with light allows performing the process in which the reduction reaction for reducing the carbon dioxide and the biochemical reaction (enzymatic reaction) for generating a substance using the formic acid which is obtained by reducing carbon dioxide (CO₂) are linked, which allows producing the high value-added substance [m].

Hereinafter, the configuration of the enzyme reactor according to the second embodiment will be described in detail.

### 1. Semipermeable Membrane

The semipermeable membrane is a membrane that partitions the above-described cathode chamber into the photocatalytic reaction chamber and the enzymatic reaction chamber, and allows the reduced carbon dioxide (the substance which is obtained by reducing carbon dioxide) to permeate through the membrane and does not allow the enzyme to permeate through the membrane.

The molecular weight cutoff (MWCO) of the semipermeable membrane is not particularly limited as long as the semipermeable membrane allows the reduced carbon dioxide to permeate through the membrane and does not allow the enzyme to permeate through the membrane. For example, it is preferably within a range of 1000 Da or more and 10000 Da or less. This is because the semipermeable membrane is preferably made into a membrane that allows the reduced carbon dioxide to permeate through the membrane and does not allow the enzyme to permeate through the membrane. The material of the semipermeable membrane is the same as the material of the semipermeable membrane according to the first embodiment, and therefore, the explanation thereof will be omitted.

### 2. Anodic electrolytic solution, Cathodic electrolytic solution, and Enzyme Reaction Solution

The anodic electrolytic solution is an electrolytic solution in which an electrolyte is dissolved in a solvent. The cathodic electrolytic solution is not particularly limited as long as the cathodic electrolytic solution is an electrolytic solution in which a carbon dioxide is dissolved in the electrolytic solution. However, the cathodic electrolytic solution is usually an electrolytic solution in which a carbon dioxide is dissolved in the common (same) electrolytic solution with the anodic electrolytic solution. The enzyme reaction solution is not particularly limited as long as the enzyme reaction solution is a solution in which an enzyme is added to a liquid such as water. The enzyme reaction solution is, for example, a solution in which an enzyme is added to the common electrolytic solution with the anodic electrolytic solution.

The common electrolytic solution used for the anodic electrolytic solution, the cathodic electrolytic solution, and the enzyme reaction solution is not particularly limited as long as the common electrolytic solution is an electrolytic solution that allows a target reduction reaction in the cathodic electrolytic solution and a target enzyme reaction in the enzyme reaction solution to be linked and performed. However, the common electrolytic solution is, for example, an electrolytic solution in which an electrolyte is dissolved in water (solvent), and among them, a mixed aqueous solution (buffer solution) of a weak acid or a weak base and a salt thereof, pH of which is adjusted within a range near the optimum pH of the enzyme, and a temperature is adjusted within a range near the optimum temperature of the enzyme.

The concentration [ppm] of carbon dioxide in the cathodic electrolytic solution is not particularly limited as long as the concentration allows the target reduction reaction in the cathodic electrolytic solution and the target enzyme reaction in the enzyme reaction solution to be linked and performed. The concentration may be appropriately selected according to the type, etc. of the electrolytic solution, the type, etc. of the enzyme reaction, and the type, concentration, etc. of the enzyme.

The enzyme contained in the enzyme reaction solution is not particularly limited as long as the enzyme is a molecule which functions as a catalyst for the target enzyme reaction in the enzyme reaction solution, and the enzyme may be appropriately selected according to the target enzyme reaction in the enzyme reaction solution. Examples of the enzyme include an oxidoreductase, a transferase, a hydrolase, a lyase, an isomerase, and a ligase (synthase), and among them, for example, an enzyme using the electron mediator as the coenzyme contained in the cathodic electrolytic solution is preferred.

The concentration [U/mL] of the enzyme in the enzyme reaction solution is not particularly limited as long as the concentration allows the target reduction reaction in the cathodic electrolytic solution and the target enzyme reaction in the enzyme reaction solution to be linked and performed. The concentration may be appropriately selected according to the conditions including the type, etc. of the electrolytic solution, the concentration, etc. of carbon dioxide, the type, etc. of the enzyme reaction, and the type, concentration, etc. of the enzyme.

### 3. Anodic Electrode, Cathodic Electrode, and Ion Exchange Membrane

The anodic electrode and the cathodic electrode are the same as those of the anodic electrode and the cathodic electrode according to the first embodiment, and therefore will not be described here. The ion exchange membrane is the same as the ion exchange membrane according to the first embodiment, and therefore will not be described here.

### 4. Enzyme Reactor

The enzyme reactor according to the second embodiment includes: the reaction vessel; the ion exchange membrane; the semipermeable membrane; the anodic electrolytic solution; the cathodic electrolytic solution; the enzyme reaction solution; the anodic electrode; and the cathodic electrode. The cathodic electrolytic solution and the enzyme reaction solution are in contact via the semipermeable membrane.

### (1) Other configurations

The enzyme reactor according to the second embodiment is not particularly limited as long as the enzyme reactor is as described above. However, the enzyme reactor usually further includes a light source similarly to the enzyme reactor according to the first embodiment. The light source is the same as the enzyme reactor according to the first embodiment.

Similarly to the enzyme reactor according to the first embodiment, the enzyme reactor further includes a reference electrode, and is preferably a device capable of measuring the potential of the cathodic electrode with respect to the potential of the reference electrode.

Similarly to the enzyme reactor according to the first embodiment, the enzyme reactor preferably further includes an external power supply.

Preferably, similarly to the enzyme reactor according to the first embodiment, the enzyme reactor includes the dialysis cup and the mounting port that allows mounting and removing of the dialysis cup at a portion of the cathode chamber side in the upper portion of the reaction vessel. A part of the dialysis cup is preferred to be the semipermeable membrane that partitions the cathode chamber into the photocatalytic reaction chamber and the enzymatic reaction chamber.

Preferably, similarly to the enzyme reactor the first embodiment, in the enzyme reactor, the enzymatic reaction chamber is partitioned and multi-layered into the plurality of enzymatic reaction chambers by the one or more semipermeable membranes, the plurality of types of enzyme reaction solutions housed in the respective plurality of enzymatic reaction chambers contain the enzymes. This is because the multi-stage enzymatic reaction is allowed.

Among the above-described devices, the enzyme reactor is preferred to be a device in which the reaction pathway is configured to be a desired pathway by configuring the permeabilities of the respective plurality of substances produced by the enzymatic reactions at the plurality of enzymatic reaction chambers for the semipermeable membrane between the photocatalytic reaction chamber and the enzymatic reaction chambers and the one or more semipermeable membrane between the enzymatic reaction chambers. This is because the complicated reaction pathway is allowed.

Among the above-described devices in which the enzymatic reaction chambers are partitioned and multi-layered into the plurality of enzymatic reaction chambers, the enzyme reactor is preferred to be a device in which the semipermeable membrane between the photocatalytic reaction chamber and the enzymatic reaction chamber and the one or more semipermeable membrane between the plurality of the enzymatic reaction chamber have different amounts (for example, different amounts within the range of 1000 Da or more and 10000 Da or less) of molecular weight cutoff (MWCO).

### (2) Application

The enzyme reactor according to the second embodiment is used in the method for producing the substance from the carbon dioxide by irradiating the photocatalyst layer of the cathodic electrode with light to allows the reduction reaction (sometimes referred to as "target reduction reaction in the cathodic electrolytic solution" in the second embodiment) in which the carbon dioxide is reduced in the cathodic electrolytic solution and the enzyme reaction (sometimes referred to as "target enzyme reaction in the enzyme reaction solution" in the second embodiment) in which the enzyme is caused to function in the enzyme reaction solution to produce the substance from the reduced carbon dioxide (the substance which is obtained by reducing carbon dioxide), to be linked and performed.

### Examples

Hereinafter, the present invention will be described in detail with reference to Examples. The present invention is not limited to the examples.

### Example 1

First, the configuration of the enzyme reactor of Example 1 will be described.

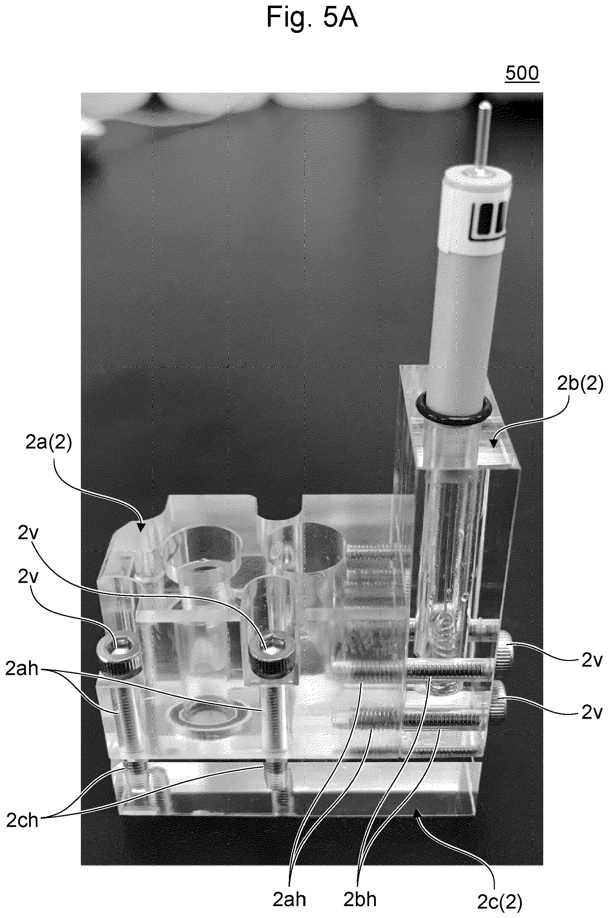

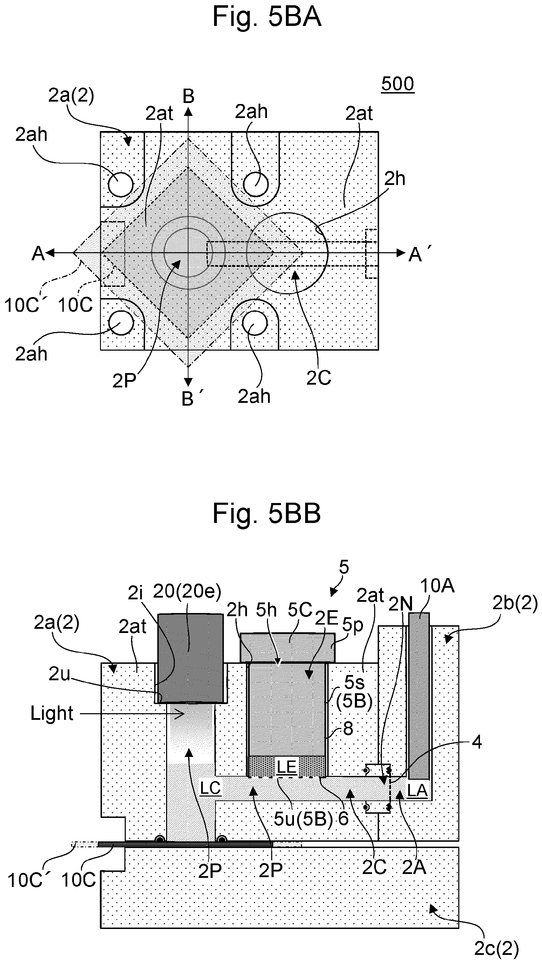

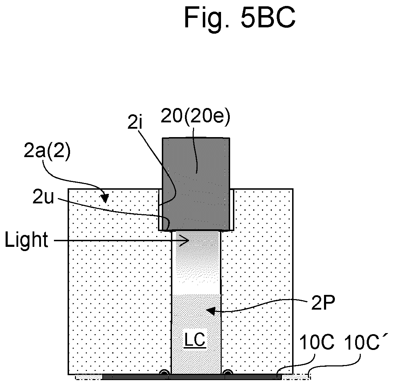

Fig. 5A is a photograph illustrating an overall outline of the enzyme reactor of Example 1. Fig. 5BA is a top view schematically illustrating a main part of the enzyme reactor of Example 1. Fig. 5BB is a cross-sectional view schematically illustrating a main part of the enzyme reactor of Example 1 and is a diagram illustrating a cross-section along the A-A' line of Fig. 5BA. Fig. 5BC is a cross-sectional view schematically illustrating a main part of the enzyme reactor of Example 1 and is a diagram illustrating a cross-section along the B-B' line of Fig. 5BA.

As illustrated in Figs. 5A and 5BA to 5BC, an enzyme reactor 500 of Example 1 includes the reaction vessel 2 including a first member 2a, a second member 2b, and a third member 2c; a proton exchange membrane (ion exchange membrane) 4; and a dialysis cup 5. The first member 2a and the second member 2b of the reaction vessel 2 are fastened by bolts 2v inserted into bolt insertion holes 2ah of the first member 2a and bolt insertion holes 2bh of the second member 2b. The first member 2a and the third member 2c of the reaction vessel 2 are fastened by bolts 2v inserted into bolt insertion holes 2ah of the first member 2a and bolt insertion holes 2ch of the third member 2c. The proton exchange membrane 4 is fixed to the first member 2a side of the second member 2b, and partitions an inside 2N of the reaction vessel 2 into an anode chamber 2A and a cathode chamber 2C. In the reaction vessel 2, the mounting port (opening) 2h having the same shape and size in plan view as those of the bottom portion 5u of the main body portion 5B of the dialysis cup 5 is provided on an upper portion 2at of the first member 2a. The dialysis cup 5 includes the cylindrical main body portion 5B, which includes a bottom portion 5u and a side portion 5s and is provided with an opening portion 5h on an upper portion thereof facing the bottom portion 5u, and a cover portion 5C (which is closed in the figure) provided to allow opening and closing the opening portion 5h of the main body portion 5B. The cover portion 5C includes a protruding portion 5p protruding laterally with respect to the side portion 5s of the main body portion 5B. The dialysis cup 5 is mounted on the mounting port 2h of the first member 2a by inserting the main body portion 5B into the cathode chamber 2C through mounting port 2h of the first member 2a and supporting the protruding portion 5p of the cover portion 5C at a periphery portion of the mounting port 2h on the upper portion 2at of the first member 2a. The dialysis cup 5 is allowed to be mounted on and removed from the mounting port 2h of the first member 2a. The bottom portion 5u of the main body portion 5B of the dialysis cup 5 is made of a semipermeable membrane 6, and the side portion 5s of the main body portion 5B is made of a non-permeable membrane 8. The cathode chamber 2C is partitioned into a photocatalytic reaction chamber 2P outside the dialysis cup 5 and an enzymatic reaction chamber 2E inside the dialysis cup 5 by the semipermeable membrane 6 of the bottom portion 5u and the non-permeable membrane 8 of the side portion 5s of the main body portion 5B of the dialysis cup 5.

The enzyme reactor 500 further includes an anodic electrolytic solution LA housed in the anode chamber 2A, a cathodic electrolytic solution LC housed in the photocatalytic reaction chamber 2P of the cathode chamber 2C, and an enzyme reaction solution LE housed in the enzymatic reaction chamber 2E of the cathode chamber 2C.

The anodic electrolytic solution LA is a predetermined electrolytic solution in which a predetermined electrolyte is dissolved in water. The cathodic electrolytic solution LC is a solution of the electron mediator dissolved in the common (same) electrolytic solution with the anodic electrolytic solution LA. The enzyme reaction solution LE is obtained by adding an enzyme and a raw material substrate to a common electrolytic solution with the anodic electrolytic solution LA. As the common electrolytic solution, specifically, for example, a mixed aqueous solution (buffer solution) of a weak acid or a weak base and a salt thereof, pH of which is adjusted within a range near the optimum pH of the enzyme, and the temperature is adjusted within a range near the optimum temperature of the enzyme, is employed.

The anodic electrolytic solution LA and the cathodic electrolytic solution LC are in contact with one another via the proton exchange membrane 4. The cathodic electrolytic solution LC and the enzyme reaction solution LE are in contact with one another via the semipermeable membrane 6. The semipermeable membrane 6 is a membrane that allows the reduced electron mediator (the substance which is obtained by reducing the electron mediator) to permeate through the membrane and does not allow the enzyme to permeate through the membrane. The non-permeable membrane 8 is a membrane that does not allow any component of the cathodic electrolytic solution LC or the enzyme reaction solution LE to permeate through the membrane.

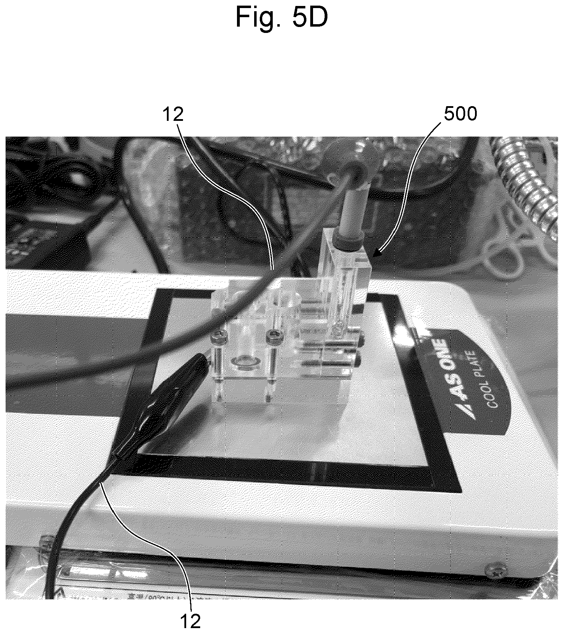

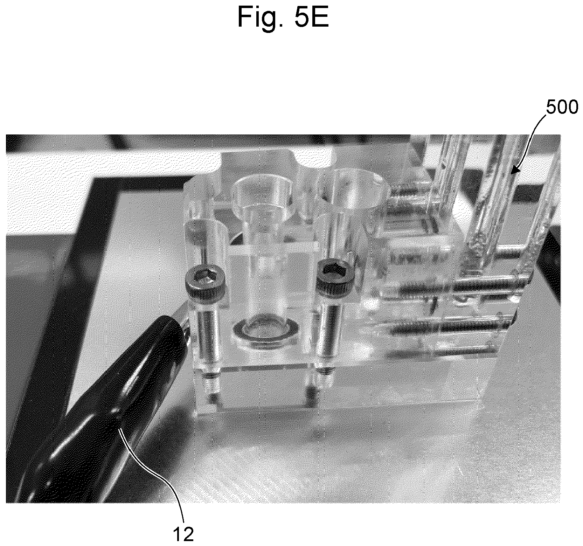

The enzyme reactor 500 further includes an anodic electrode 10A arranged in the anode chamber 2A to be immersed in the anodic electrolytic solution LA and a cathodic electrode 10C. The cathodic electrode 10C includes a cathode substrate made of a copper substrate and a photocatalyst layer formed on an upper surface of the cathode substrate. The cathodic electrode 10C is fixed between the first member 2a and the third member 2c, and is disposed on the photocatalytic reaction chamber 2P such that the upper surface of the photocatalyst layer is immersed in the cathodic electrolytic solution LC. The enzyme reactor 500 further includes an external circuit (illustrated in Fig. 5D and Fig. 5E below) including a wiring. The wiring electrically connects the anodic electrode 10A to the cathodic electrode 10C. The enzyme reactor 500 further includes a light source 20 disposed on the photocatalytic reaction chamber 2P so as to face the cathodic electrode 10C, and a power supply for light source (not illustrated) disposed outside the reaction vessel 2 and connected to the light source 20. Specifically, the light source 20 is disposed on the photocatalytic reaction chamber 2P by inserting a cylindrical tip portion 20e, which irradiates light, into the photocatalytic reaction chamber 2P through a light source insertion hole 2i of the reaction vessel 2 and supporting the cylindrical tip portion 20e at a stepped portion 2u of the light source insertion hole 2i. Specifically, a halogen lamp is used as the light source 20.

Next, a method for using the enzyme reactor of Example 1 will be described.

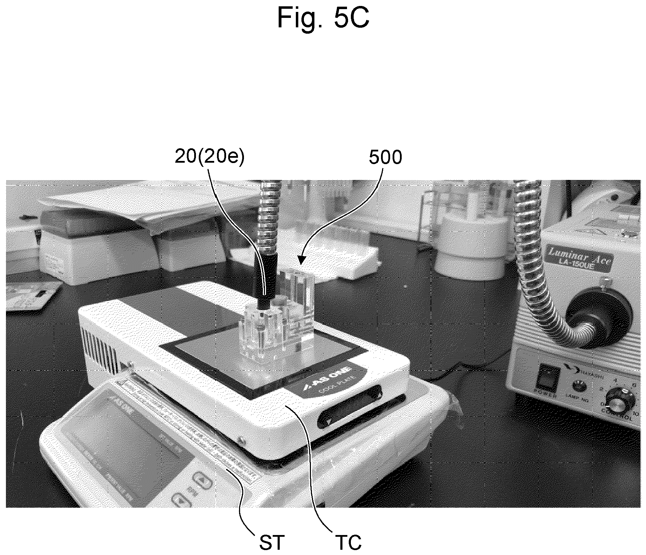

Fig. 5C is a photograph illustrating an aspect of the method for using the enzyme reactor of Example 1. Furthermore, Fig. 5D is a photograph illustrating another aspect of the method for using the enzyme reactor of Example 1, and Fig. 5E is a photograph obtained by enlarging a part of the device photographed in the photograph of Fig. 5D.

In the method for using the enzyme reactor 500 of Example 1, as illustrated in Fig. 5C, supplying power to the light source 20 from the power supply for light source (not illustrated) allows the light source 20 to irradiate the photocatalyst layer of the cathodic electrode with light through the cathodic electrolytic solution LC with a light in, for example, the wavelength band from 350 nm to 3500 nm. Furthermore, in the method for using the enzyme reactor 500, as illustrated in 5D and 5E, while the light source 20 irradiates the photocatalyst layer of the cathodic electrode 10C with light, the positive voltage and the negative voltage may be applied to the anodic electrode 10A and the cathodic electrode 10C by the external power supply (not illustrated) via the wiring 12, respectively.

Further, in the method for using the enzyme reactor 500, as illustrated in Fig. 5C, a temperature controller TC may be used, and the light source 20 may irradiate the photocatalyst layer of the cathodic electrode 10C with light while the reaction vessel 2 is heated or cooled. As a result, the target reaction can be caused in the enzyme reaction solution LE while a temperature of the enzyme reaction solution LE of the enzymatic reaction chamber 2E inside the dialysis cup 5 is adjusted to be within a desired range. In the method for using the enzyme reactor 500, as illustrated in Fig. 5C, a stirrer ST may be used, and the light source 20 may irradiate the photocatalyst layer of the cathodic electrode 10C with light while a stirrer (not illustrated) in the enzyme reaction solution LE of the enzymatic reaction chamber 2E inside the dialysis cup 5 is rotated. This allows the enzyme reaction solution LE to be agitated to improve the efficiency of the target response in the enzyme reaction solution LE.

In the enzyme reactor 500 of Example 1, another cathodic electrode 10C' is allowed to be arranged in the photocatalytic reaction chamber 2P such that an upper surface of the photocatalyst layer thereof is immersed in the cathodic electrolytic solution LC by loosening the bolts 2v inserted into the bolt insertion holes 2ah of the first member 2a and the bolt insertion holes 2ch of the third member 2c, removing the cathodic electrode 10C, arranging the other cathodic electrode 10C' between the first member 2a and the third member 2c, and then fastening the bolts 2v again. Therefore, in the single the reaction vessel 2, an optimum photocatalyst layer for performing a process in which a reduction reaction and a biochemical reaction (enzymatic reaction) are linked can be searched by replacing and using a plurality of cathodic electrodes having mutually different photocatalyst layers. In addition, even when an amount of the enzyme reaction solution LE housed in the enzymatic reaction chamber 2E inside the dialysis cup 5 is very small, a process in which a reduction reaction and a biochemical reaction (enzymatic reaction) are linked is allowed to be performed, thus saving the screening cost.

### Example 2

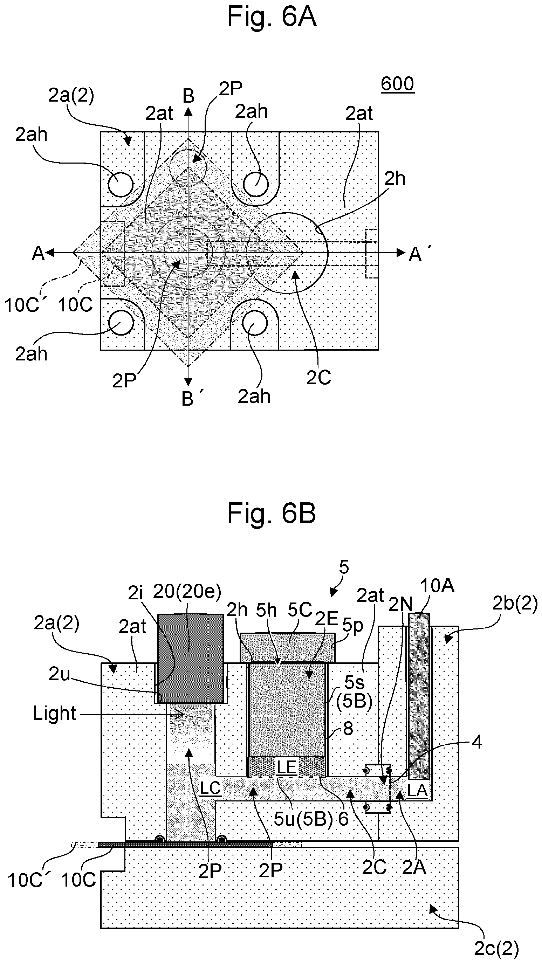

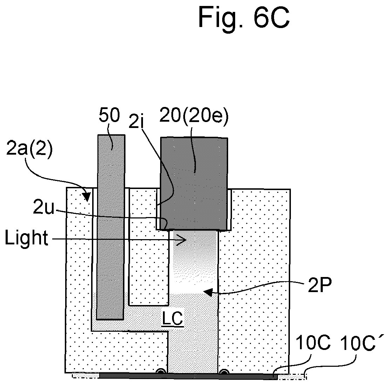

The enzyme reactor of Example 2 will be described mainly in terms of differences from the enzyme reactor of Example 1. Fig. 6A is a top view schematically illustrating a main part of the enzyme reactor of Example 2. Fig. 6B is a cross-sectional view schematically illustrating a main part of the enzyme reactor of Example 2, and is a diagram illustrating a cross section taken along the A-A' line in Fig. 6A. Fig. 6C is a cross-sectional view schematically illustrating a main part of the enzyme reactor of Example 2, and is a diagram illustrating a cross section taken along the B-B' line in Fig. 6A.

An enzyme reactor 600 of Example 2 includes the reference electrode 50 and the external circuit (not illustrated) including the wiring, the external power supply, the ammeter, and the voltmeter, instead of the external circuit included in the enzyme reactor 500 of Example 1. The wiring electrically connects the anodic electrode 10A to the cathodic electrode 10C. The external power supply applies the positive voltage and the negative voltage to the anodic electrode and the cathodic electrode via the wiring, respectively. The ammeter is connected to the wiring and measures the current flowing between the anodic electrode and the cathodic electrode. The reference electrode 50 is arranged so as to be immersed in the cathodic electrolytic solution LC in the vicinity of the cathodic electrode 10C in the photocatalytic reaction chamber 2P, and is connected to the negative electrode of the external power supply by the wiring. The voltmeter is connected to the reference electrode 50 and the negative electrode of the external power supply by the wiring, and measures the potential of the cathodic electrode 10C with respect to the potential of the reference electrode 50. The configuration of the enzyme reactor 600 of Example 2 is the same as that of the enzyme reactor 500 of Example 1 except for the above-described points.

In the method for using the enzyme reactor 600 of Example 2, similarly to the method for using the enzyme reactor 500 of Example 1, the light source 20 irradiates the photocatalyst layers 10Cp of the cathodic electrode 10C with light through the cathodic electrolytic solution LC. At the same time, the external power supply applies the positive voltage and the negative voltage to the anodic electrode 10A and the cathodic electrode 10C, respectively. At this time, the potential of the cathodic electrode 10C with respect to the potential of the reference electrode 50 is set to a predetermined value such that the total voltage of the voltage generated by irradiating the photocatalyst layer 10Cp with light and the voltage applied by the external power supply 34 is equal to or higher than the voltage required for causing the target reduction reaction in the cathodic electrolytic solution LC to occur.

The present invention is not limited to the above-described embodiment, and includes various modifications. For example, the above-described embodiments have been described in detail to describe the present invention in an easy-to-understand manner, and are not necessarily limited to all the above-described configurations. Further, it is possible to replace a part of the configuration of one example with the configuration of another example, and it is also possible to add the configuration of another example to the configuration of one example. Further, it is possible to add, delete, or replace a part of the configuration of each example with other configuration.

### Reference Signs List

- 2: Reaction vessel
- 2A: Anode chamber
- 2C: Cathode chamber
- 2P: Photocatalytic reaction chamber
- 2E: Enzymatic reaction chamber
- 4: Proton exchange membrane (ion exchange membrane)
- 5: Dialysis cup
- 6: Semipermeable membrane
- 8: Non-permeable membrane
- 10A: Anodic electrode
- 10C: Cathodic electrode
- 10Ca: Cathode substrate
- 10Cp: Photocatalyst layer
- LA: Anodic electrolytic solution
- LC: Cathodic electrolytic solution
- LE: Enzyme reaction solution
- 100: Enzyme reactor
- 200: Enzyme reactor
- 300: Enzyme reactor
- 400: Enzyme reactor
- 500: Enzyme reactor
- 600: Enzyme reactor

## Claims

1. An enzyme reactor (100) comprising:
a reaction vessel (2);
an ion exchange membrane (4) that partitions an inside of the reaction vessel (2) into an anode chamber (2A) and a cathode chamber (2C);
a semipermeable membrane (6) that partitions the cathode chamber (2C) into a photocatalytic reaction chamber (2P) and an enzymatic reaction chamber (2E);
an anodic electrolytic solution (LA) housed in the anode chamber (2A);
a cathodic electrolytic solution (LC) housed in the photocatalytic reaction chamber (2P);
an enzyme reaction solution (LE) housed in the enzymatic reaction chamber (2E);
an anodic electrode (10A) disposed in the anode chamber (2A) so as to be immersed in the anodic electrolytic solution (LA); and
a cathodic electrode (10C) disposed in the photocatalytic reaction chamber (2P) so as to be immersed in the cathodic electrolytic solution (LC),
wherein the cathodic electrode (10C) has a surface on which a photocatalyst layer (10Cp) containing a photocatalyst is disposed,
wherein the cathodic electrolytic solution (LC) contains an electron mediator,
wherein the enzyme reaction solution (LE) contains an enzyme and a raw material substrate,
wherein the cathodic electrolytic solution (LC) and the enzyme reaction solution (LE) are in contact with one another via the semipermeable membrane (6), and
wherein the semipermeable membrane (6) allows the reduced electron mediator to permeate through the semipermeable membrane (6) and does not allow the enzyme to permeate through the semipermeable membrane (6).

2. An enzyme reactor (100) comprising:
a reaction vessel (2);
an ion exchange membrane (4) that partitions an inside of the reaction vessel (2) into an anode chamber (2A) and a cathode chamber (2C);
a semipermeable membrane (6) that partitions the cathode chamber (2C) into a photocatalytic reaction chamber (2P) and an enzymatic reaction chamber (2E);
an anodic electrolytic solution (LA) housed in the anode chamber (2A);
a cathodic electrolytic solution (LC) housed in the photocatalytic reaction chamber (2P);
an enzyme reaction solution (LE) housed in the enzymatic reaction chamber (2E);
an anodic electrode (10A) disposed in the anode chamber (2A) so as to be immersed in the anodic electrolytic solution (LA); and
a cathodic electrode (10C) disposed in the photocatalytic reaction chamber (2P) so as to be immersed in the cathodic electrolytic solution (LC),
wherein the cathodic electrode (10C) has a surface on which a photocatalyst layer (10Cp) containing a photocatalyst is disposed,
wherein the cathodic electrolytic solution (LC) contains carbon dioxide,
wherein the enzyme reaction solution (LE) contains an enzyme,
wherein the cathodic electrolytic solution (LC) and the enzyme reaction solution (LE) are in contact with one another via the semipermeable membrane (6), and
wherein the semipermeable membrane (6) allows the reduced carbon dioxide to permeate through the semipermeable membrane (6) and does not allow the enzyme to permeate through the semipermeable membrane (6).

3. The enzyme reactor (100) according to claim 1 or 2, wherein
the semipermeable membrane (6) has a molecular weight cutoff (MWCO) within a range of 1000 Da or more and 10000 Da or less.

4. The enzyme reactor (100) according to claim 1 or 2, further comprising
an external power supply that applies a positive voltage and a negative voltage to the anodic electrode (10A) and the cathodic electrode (10C), respectively.
